# FASCICULE DE BREVET EUROPEEN

(11) **EP 0 643 701 B1**
(45) Date de publication et mention de la délivrance du brevet: **05.12.2001**
(21) Numéro de dépôt: 92911098.9
(22) Date de dépôt: 29.05.1992
(51) Int. Cl.: C07D 235/18, C07F 9/6506, A61K 31/415, A61K 7/40

(54) **COMPOSES DERIVES DE BENZIMIDAZOLE, LEUR PROCEDE DE PREPARATION ET LEUR UTILISATION DANS LES DOMAINES THERAPEUTIQUE ET COSMETIQUE**
BENZIMIDAZOL-DERIVATE, VERFAHREN ZU IHRER HERSTELLUNG UND IHRE ANWENDUNG IN DEN GEBIETEN THERAPEUTIK UND KOSMETIK
BENZIMIDAZOLE-DERIVED COMPOUNDS, METHOD FOR PREPARING SAME, AND THERAPEUTICAL AND COSMETIC USES THEREOF

(30) Priorité: 31.05.1991 FR 9106583
(43) Date de publication de la demande: 22.03.1995
(73) Titulaire: CENTRE INTERNATIONAL DE RECHERCHES DERMATOLOGIQUES GALDERMA, ( CIRD GALDERMA), 06560 Valbonne (FR)
(72) Inventeur: ROCHER, Jean-Philippe, F-74240 Gaillard (FR); CAVEY, Daniel, F-06560 Valbonne (FR)
(74) Mandataire: Stalla-Bourdillon, Bernard
(86) Numéro de dépôt international: FR9200482
(87) Numéro de publication internationale: WO9221663

(56) Documents cités:
- EP-A- 0 199 636
- GB-A- 2 164 648

## Description

La présente invention a pour objet de nouveaux dérivés du benzimidazole, leur procédé de préparation et leur utilisation dans les domaines thérapeutique et cosmétique.

Ces nouveaux dérivés du benzimidazole se sont avérés présenter en médecine humaine ou vétérinaire une bonne activité à l'égard des états inflammatoires et/ou immunoallergiques.

En cosmétique, ces nouveaux dérivés du benzimidazole constituent des substances particulièrement intéressantes dans l'hygiène corporelle et capillaire.

L'état de la technique concernant les dérivés du benzimidazole est essentiellement représenté par la demande de brevet français n° 85 13747 (2.570.377) qui décrit certains dérivés du benzimidazole présentant un profil biologique les apparentant aux composés connus sous la dénomination de "rétinoïde" et ayant une activité renforcée dans le traitement des affections dermatologiques liées à un désordre de la kératinisation (différenciation-prolifération).

Les dérivés du benzimidazole selon l'invention peuvent être représentés par la formule générale suivante : 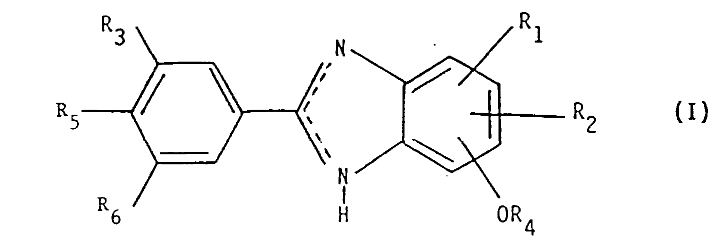 dans laquelle :
R₁ et R₂ représentent un atome d'hydrogène, un radical alkyle inférieur, un radical OR₄, un radical fluoroalkyle inférieur ou un atome d'halogène,
R₃ représente un atome d'hydrogène, un radical alkyle inférieur, un halogène, un hydroxyle ou un radical alcoxy inférieur ayant de 1 à 6 atomes de carbone,
R₄ représente un atome d'hydrogène, un radical alkyle inférieur, un radical benzyle, ou un radical 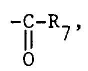 PO₃H, SO₃H ou un reste d'acide aminé,
R₇ représente un radical alkyle inférieur, un radical alcoxy inférieur ayant de 1 à 6 atomes de carbone, le radical -(CH₂)ₙ-COOH, n = 1 à 6, ou le radical 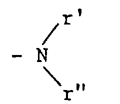
r' et r" représentant un atome d'hydrogène, un radical alkyle inférieur, ou r' et r" pris ensemble forment avec l'atome d'azote, un hétérocyle à 5 ou 6 chaînons,
R₅ et R₆, représentent l'un, un radical OR₈ et l'autre un radical cycloalkyle mono ou polycyclique ayant de 5 à 12 atomes de carbone, lié au noyau phényle par un carbone tertiaire ;
R₈ représente un atome d'hydrogène, un radical alkyle inférieur, un radical acyle ayant de 2 à 7 atomes de carbone, un radical benzyle éventuellement substitué par un ou plusieurs atomes d'halogène ou le radical benzoyle,
et les sels desdits composés obtenus par addition d'un acide ou d'une base pharmaceutiquement acceptable.

Selon l'invention, les sels sont obtenus par addition d'une base lorsque R₄ représente SO₃H, PO₃H, ou -CO(CH₂)ₙ-COOH. Ces sels peuvent également être obtenus par addition d'un acide lorsque R₅ représente le radical 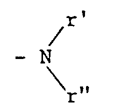 r' et r" pris ensemble formant un hétérocycle basique.

Il est également possible de former des sels par addition d'un acide sur les atomes d'azote du noyau benzimidazole.

Par radical alkyle inférieur, on doit entendre un radical linéaire ou ramifié ayant de 1 à 6 atomes de carbone, notamment un radical méthyle, éthyle, isopropyle, butyle, 1-méthylpropyle, 1-éthylpropyle, tertiobutyle, 1,1-diméthylpropyle et 1-méthyl 1-éthylpropyle,

Par radical fluoroalkyle inférieur, on doit entendre un radical ayant de 1 à 6 atomes de carbone et de 1 à 5 atomes de fluor tel que le radical trifluorométhyle.

L'atome d'halogène est selon l'invention de préférence un atome de chlore, brome ou fluor.

Par radical alcoxy inférieur ayant de 1 à 6 atomes de carbone, on doit entendre notamment un radical méthoxy, éthoxy, isopropoxy ou butoxy.

Par radical cycloalkyle mono ou polycyclique, ayant de 5 à 12 atomes de carbone, lié au noyau phényle par un carbone tertiaire, on doit entendre notamment les radicaux 1-adamantyle ou 1-méthylcyclohexyle.

Par radical acyle, ayant de 2 à 7 atomes de carbone, on doit entendre notamment, les radicaux acétyle, propionyle, butyryle, isobutyryle, valéryle ou pivalyle.

Lorsque r' et r" pris ensemble forment avec l'atome d'azote un hétérocycle à 5 ou 6 chaînons, celui-ci est un radical pipéridino, morpholino, pyrrolidino, ou pipérazino, éventuellement substitué en position 4 par un radical alkyle inférieur.

Par reste d'acide aminé, on doit entendre plus particulièrement les restes dérivant de lysine, de glycine ou de l'acide aspartique, ceux-ci étant liés au noyau benzénique par l'intermédiaire d'une fonction ester.

Parmi les composés de formule (I) ci-dessus, on peut notamment citer les suivants :
4-hydroxy-2-[3-(1-adamantyl)-4-hydroxyphényl] benzimidazole,
5-hydroxy-2-[3-(1-adamantyl)-4-hydroxyphényl] benzimidazole,
5-benzyloxy-2-[3-(1-adamantyl)-4-méthoxyphényl] benzimidazole,
5-hydroxy-2- [3-(1-adamantyl)-4-méthoxyphényl] benzimidazole,
5-benzyloxy-2- [3-(1-méthylcyclohexyl)-4-benzyloxyphényl] benzimidazole,
5-hydroxy-2-[3-(1-méthylcyclohexyl)-4-hydroxyphényl] benzimidazole,
5-méthoxy-2-[3-(1-adamantyl)-4-benzyloxyphényl] benzimidazole,
5-méthoxy-2- [3-(1-adamantyl)-4-hydroxyphényl] benzimidazole,
5-benzyloxy-2- [3-benzyloxy-4-(1-adamantyl)phényl] benzimidazole,
5-hydroxy-2-[3-hydroxy-4-(1-adamantyl)phényl] benzimidazole,
5-benzyloxy-2-[3-(1-adamantyl)-4-benzyloxy-5-méthoxyphényl] benzimidazole,
5-hydroxy-2-[3-(1-adamantyl)-4-hydroxy-5-méthoxyphényl] benzimidazole,
5-benzyloxy-2-[3-(1-adamantyl)-4-benzyloxyphényl] benzimidazole,
5-méthoxy-2-[3-(1-adamantyl)-4-méthoxyphényl] benzimidazole,
5-acétoxy-2-[3-(1-adamantyl)-4-acétoxyphényl]benzimidazole,
5-acétoxy-2- [3-(1-adamantyl)-4-hydroxyphényl]benzimidazole.

La présente invention a également pour objet le procédé de préparation des composés de formule (I).

Diverses méthodes de synthèse permettent d'accéder aux composés de formule (I) mais l'on préfère selon l'invention utiliser les méthodes suivantes :

Selon la première méthode, on réalise la condensation d'un dérivé d'acide carboxylique aromatique de formule (1) sur une orthodiamine aromatique de formule (2) suivant le schéma (A) réactionnel ci-dessous :

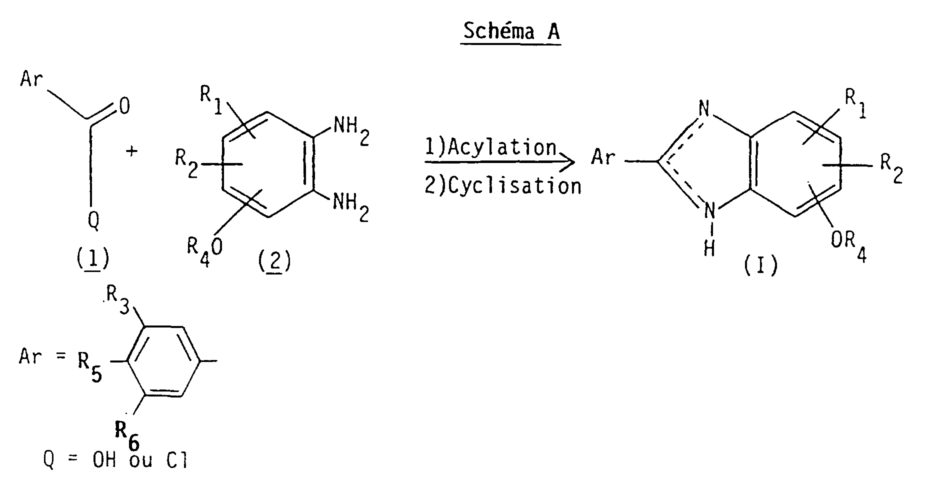

La réaction d'acylation est réalisée de manière classique en utilisant le composé de formule (1) sous forme de chlorure d'acide en présence d'une amine tertiaire dans le THF.

La réaction de cyclisation est effectuée par chauffage à reflux sous atmosphère d'azote du mélange réactionnel en présence d'un solvant aromatique tel que le toluène ou le xylène en utilisant un réactif tel que l'acide paratoluène sulfonique ou l'oxychlorure de phosphore.

Selon une seconde méthode plus particulièrement préférée, on fait réagir, dans un premier temps, un dérivé d'acide carboxylique aromatique de formule (1) qui est solubilisé dans le dichlorométhane ou dans le toluène sur une orthonitroaniline de formule (3) dans la pyridine à la température de reflux du solvant présentant le point d'ébullition le plus faible (40 à 100°C). Le composé intermédiaire obtenu (4) est alors soumis à une réduction par hydrogénation calytique (Palladium sur charbon à 5 %) ou en utilisant un sel métallique tel que le chlorure d'étain dans le THF puis à une cyclisation comme indiqué précédemment pour la méthode selon le schéma A.

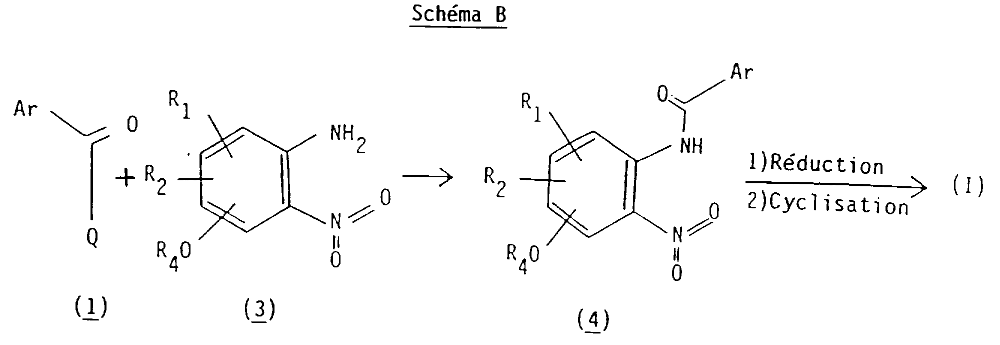

Lorsque les substituants de la partie aromatique et/ou de la partie hétérocylique du composé de formule (I) sont des fonctions hydroxyles, une protection préalable de cette fonction doit être réalisée sous forme d'ester, de dérivé alcoxy ou benzyloxy. Dans ces cas, après la réaction de cyclisation, une étape supplémentaire de déprotection est nécessaire. Elle peut s'effectuer par hydrogénolyse en présence d'un catalyseur au palladium dans le cas d'un substituant benzylique, par un acide de Lewis tel que AlCl₃, BBr₃ ou par le chlorhydrate de pyridine dans le cas d'un substituant alkyle ou benzyle, par saponification si la fonction phénolique est protégée sous forme d'ester. Les méthodes de déprotection catalysées par les métaux de transition comme le palladium ou le rhodium peuvent également être utilisées.

Les fonctions hydroxyles peuvent être transformées en esters, esters carbamiques, esters sulfoniques, esters phosphoniques, ou alkylées suivant des procédés classiques ; ces analogues correspondent aux différentes significations données au radical R₄.

La présente invention a également pour objet à titre de médicament, les composés de formule générale (I) tels que définis ci-dessus.

Les composés selon l'invention présentent en particulier une bonne activité dans les tests :
a) d'inhibition de l'inflammation (oedème de l'oreille) induite par l'application topique d'acide arachidonique chez la souris,
b) d'inhibition de l'allergie de contact (oedème de l'oreille) induite par l'application topique d'oxazolone chez la souris.

Ces tests sont respectivement admis comme mesure d'activité anti-inflammatoire et d'activité anti-inflammatoire immuno-dépendante.

Les composés selon l'invention trouvent tout particulièrement une application en médecine humaine ou vétérinaire pour la préparation de médicaments destinés au traitement ou à la prévention des états inflammatoires et/ou immuno-allergiques.

Ces médicaments conviennent particulièrement pour traiter les dermatites atopiques, les dermites séborrhéïues, les dermites dues à des irritants , les névrodermites, les eczémas d'origines diverses, l'érythème fessier du nourrisson, les érythroses faciales, l'acné rosacée, les ichtyoses, les psoriasis, le lichen plan, les prurigos, les piqûres d'insectes.

Ils peuvent être également utilisés dans les cas d'arthrite, de synovite, de tendinite, d'entorse et dans tous les états inflammatoires post traumatologiques ou rhumatologiques.

Ils peuvent aussi être indiqués dans le cas d'inflammation de la sphère ORL (rhinites), d'inflammation ophtalmique (conjonctivites ou eczéma des paupières par exemple), et d'inflammation des muqueuses (gingivites, stomatites, vulvites, etc...).

Les composés de l'invention peuvent également être utilisés, à titre de médicaments, comme adjuvants du traitement étiologique des dermatoses inflammatoires d'origine infectieuse ou parasitaire et comme adjuvant du traitement anti-acnéique.

Les composés selon l'invention trouvent également une application dans l'hygiène corporelle et capillaire et dans la prévention (photoprotection) et le traitement des érythèmes solaires, dans la prévention des inflammations cutanées photoinduites (éventuellement en association avec d'autres photoprotecteurs) ou encore dans la protection de la peau contre les solvants ou les irritants primaires. Les compositions selon l'invention peuvent également être utilisées sous la forme de crème anti-irritante pour peaux sensibles.

La présente invention a donc également pour objet de nouvelles compositions médicamenteuses destinées notamment au traitement des affections susmentionnées caractérisées par le fait qu'elles contiennent dans un support pharmaceutiquement acceptable au moins un composé de formule (I).

L'administration peut être effectuée par voie entérale, parentérale ou locale (topique : peau ou muqueuse ou oculaire).

Par voie entérale ou parentérale, les composés selon l'invention sont généralement administrés à une dose journalière de 0,01 à 100 mg/kg de poids corporel.

Les compositions par voie orale peuvent se présenter par exemple sous la forme de comprimés, de gélules, de dragées, de sirops, de suspensions, de solutions, de poudres, de granulés ou d'émulsions ou encore de microsphères, de nanosphères ou vésicules lipidiques ou polymériques, permettant une libération controlée du principe actif.

Les compositions par voie locale contiennent généralement entre 0,005 % et 10 % en poids de composé de formule (I) selon l'invention.

Les compositions par voie topique se présentent notamment sous la forme d'onguents, de crèmes, de laits, de pommades, de poudres, de pâtes gingivales, de tampons imbibés, de solutions, de lotions, de gels, de sprays, de shampooings, de lotions lavantes ou encore de suspensions, de microsphères ou nanosphères ou vésicules lipidiques ou polymériques, de patches polymériques ou d'hydrogels permettant une libération controlée du principe actif.

Les compositions administrées par voie oculaire sont principalement des collyres.

Les compositions administrées par voie nasale peuvent se présenter principalement sous forme d'aérosols.

La présente invention a également pour objet une nouvelle composition cosmétique destinée à prévenir ou corriger l'aspect inesthétique de la peau, en particulier provoqué par les états inflammatoires. Les compositions cosmétiques selon l'invention contiennent généralement de 0,001 à 5 % en poids de composé de formule (I).

Les compositions cosmétiques se présentent sous la même forme que les compositions pharmaceutiques pour la voie topique.

Toutes ces formes pharmaceutiques ou cosmétiques sont préparées selon les méthodes usuelles.

Les compositions médicamenteuses et cosmétiques selon l'invention peuvent contenir des additifs inertes ou même pharmacologiquement ou cosmétiquement actifs et notamment : des agents hydratants comme la thiamorpholinone et ses dérivés ou l'urée, des vitamines telles que la vitamine A, E ou F ou leurs dérivés, des agents antiséborrhéiques tels que la S-carboxyméthyl-cystéine, la S-benzylcystéamine et leurs dérivés, la tioxolone, des agents anti-acnéiques tels que l'acide rétinoïque et ses dérivés ou l'acide 6-[3-(1-adamantyl)-4-méthoxyphényl]-2-naphtoïque et ses analogues, des antibiotiques comme l'érythromycine et ses esters, la néomycine, les tétracyclines, les antifongiques tels que les polyméthylène -4,5 isothiazolinones ou les dérivés de pyrithione, des agents favorisant la repousse des cheveux, comme le "Minoxidil" (diamino-2,4 pipéridino-6 pyrimidine oxyde-3) et ses dérivés, le Diazoxide (chlorométhyl-3 benzothiadiazine-1,2,4 dioxyde-1,1), le Phénytoïn (diphényl-5,5 imidazolidinedione-2,4) et l'iodure d'oxapropanium, d'autres agents anti-inflammatoires non stéroïdiens tels que le N-benzylphényl-α-acétoxy acétamide, les dérivés d'acides gras polyinsaturés tels que l'acide eicosa-(5,8,11)-triynoïque et l'acide eicosa (5,8,11,14)-tétraynoïque, leurs esters et leurs amides, les antihistaminiques tels que la prométhazine ou la cinnarizine, les antiprurigineux tels que l'acide palmitoyl-collagénique, les agents antipsoriatiques tels que l'anthraline et ses dérivés, ou des agents photoprotecteurs tels que filtres UVA et UVB.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UVA et UVB, des antioxydants tels que l'α-tocophérol, le butylhydroxyanisole ou le butylhydroxytoluène.

### EXEMPLES DE PREPARATION

### EXEMPLE 1 : 4-Hydroxy-2-[3-(1-adamantyl)-4-hydroxyphényl] benzimidazole

### a) 2-benzyloxy-6-nitro aniline

Dans un ballon, on introduit 6,16 g (40 mM) de 2-amino-3-nitrophénol, 60 ml de diméthylformamide et 6,62 g (48 mM) de carbonate di-potassique. Puis on ajoute, goutte à goutte, 4,75 ml (40 mM) de bromure de benzyle et le mélange réactionnel est porté à 80°C. Après avoir agité sous atmosphère inerte pendant 1,5 heures, le milieu est jeté dans de l'eau glacée puis extrait par 200 ml d'acétate d'éthyle. La phase organique est séparée par décantation et après un lavage avec une solution saturée de chlorure de sodium, elle est séchée sur sulfate de magnésium et évaporée à sec. Le produit est chromatographié sur colonne de silice (éluant : acétate d'éthyle 10 % et hexane 90 %) pour conduire à 8,8 g (90 %) du composé attendu sous forme d'une huile orangée.

### b) 6-amino-2-benzyloxy aniline

Dans un ballon, sous atmosphère inerte, on introduit 7,32 g (30 mM) du produit précédent, 70 ml d'éthanol et 28,44 g (150 mM) de dichlorure d'étain. Après addition de 4,5 ml d'acide chlorhydrique concentré, le mélange est porté à reflux pendant 3 heures. Puis le milieu est refroidi et on ajoute 150 ml d'acétate d'éthyle puis 20 ml d'une solution de soude à 40 % et 100 ml d'eau. Le produit est extrait par l'acétate d'éthyle, la phase organique est lavée par de l'eau, puis par une solution saline et enfin séchée sur sulfate de magnésium. Après évaporation à sec, on obtient 5 g (77 %) d'une huile orangée qui cristallise et qui est utilisée telle quelle. F = 68°C.

### c) Ester méthylique de l'acide 3-(1-adamantyl)-4-benzyloxy benzoïque

Dans un tricol de 1000 ml maintenu sous azote et muni d'un agitateur magnétique, on introduit 28,6 g (100 mM) de l'ester méthylique de l'acide 3-(1-adamantyl)-4-hydroxy benzoïque, 41,4 g (300 mM) de carbonate di-potassique et 300 ml de méthyléthylcétone. On ajoute ensuite au mélange porté au reflux, 13,06 ml (110 mM) de bromure de benzyle. Le milieu réactionnel est agité à reflux pendant 4 heures. Après avoir refroidi, la suspension est filtrée et le précipité est lavé à l'acétone. Le filtrat est ensuite évaporé à sec et on obtient 39,8 g d'une huile qui cristallise. Ce produit est utilisé tel que pour la suite de la synthèse. F = 126-128°C.

### d) Acide 3-(1-adamantyl)-4-benzyloxy benzoïque

Le composé préparé précédemment est dissous dans 400 ml de butanol-1 et, après addition de 16,8 g (300 mM) de pastilles de potasse, le mélange est porté à reflux pendant 45 minutes. Le milieu réactionnel est ensuite évaporé à sec, puis repris par 300 ml d'eau et acidifié avec 60 ml d'acide chlorhydrique 5N. Le précipité est filtré et lavé plusieurs fois à l'eau, puis séché. On obtient 35,5 g (98,1 %) d'acide 3-(1-adamantyl) -4-benzyloxy benzoïque de point de fusion F = 251-253°C.

### e) Chlorure de l'acide 3-(1-adamantyl)-4-benzyloxy benzoïque

Dans un ballon, on introduit 13,7 g (38 mM) de l'acide obtenu ci-dessus et 140 ml de toluène et 0,1 ml de diméthylformamide. On chauffe à 80°C et on ajoute, goutte à goutte, au mélange 3 ml (41,8 mM) de chlorure de thionyle, puis on chauffe à reflux jusqu'à ce que le dégagement gazeux cesse. On évapore à sec et on obtient ainsi 14,4 g de chlorure de l'acide 3-(1-adamantyl)-4-benzyloxy benzoïque que l'on utilise tel quel pour la synthèse suivante.

### f) 2-benzyloxy-6-amino N-[3-(1-adamantyl)-4-benzyloxybenzoyl)] aniline

Dans un ballon, on introduit 4,06 g (19 mM) de 6-amino-2-benzyloxy aniline, 2,64 ml (19 mM) de triéthylamine et 30 ml de THF sec. On refroidit à 4°C et on ajoute, goutte à goutte, 7,22 g (19 mM) de chlorure d'acide précédent et solubilisé dans 60 ml de tétrahydrofuranne (THF) sec. On agite ensuite à 20°C pendant 12 heures, puis on jette le milieu réactionnel dans l'eau et après extraction par l'acétate d'éthyle, la phase organique est décantée, lavée avec une solution saline et évaporée après séchage sur sulfate de magnésium. Le résidu obtenu est chromatographié sur colonne de silice (éluant : acétate d'éthyle 20 % et hexane 80 %) pour conduire à 7,1 g (67 %) de 2-benzyloxy-6-amino-N-[3-(1-adamantyl)-4-benzyloxybenzoyl] aniline. F = 80-82°C.

### g) 4-benzyloxy-2-[3-(1-adamantyl)-4-benzyloxyphényl]benzimidazole

Dans un ballon, 6,98 g (12,5 mM) d'amide obtenu ci-dessus sont mis en suspension dans 60 ml de toluène et le milieu est chauffé à 80°C. Puis on ajoute à goutte à goutte 2,32 ml (25 mM) d'oxychlorure de phosphore. Le mélange est agité à 100°C pendant 1 heure, puis refroidi. Après addition de 50 ml d'hexane, le précipité qui s'est formé est filtré et lavé à l'éther. Le produit est passé sous forme de base dans le tétrahydrofuranne (THF) par addition d'une solution aqueuse de soude à 45 %. Après agitation, la solution est extraite par l'acétate d'éthyle, puis la phase organique est décantée, séchée et évaporée à sec. Par chromatographie sur colonne de silice (éluant : acétate d'éthyle 40 % et hexane 60 %) on obtient 4,3 g (63 %) de produit attendu. F = 109-111°C.

### h) 4-hydroxy-2-[3-(1-adamantyl)-4-hydroxyphényl]benzimidazole

Dans le réacteur d'un autoclave, on introduit 4,1 g (7,6 mM) de l'éther benzylique obtenu ci-dessus et 450 ml d'un mélange d'acide acétique 50 Z et de dioxanne 50 %. A cette solution, on ajoute 346,4 mg de palladium sur charbon à 10 % et après avoir purgé le réacteur par de l'azote, l'hydrogène est introduit jusqu'à une pression de 7 bars. Le mélange est agité à 60°C pendant 2 heures. Puis le milieu est refroidi et filtré sur célite. Après rinçage de la célite au THF, le filtrat est évaporé à sec. Après chromatographie sur colonne de silice (éluant : THF 30 % et hexane 70 %) et recristallisation dans un mélange éthanol-eau, on obtient 1,2 g (44 % de 4-hydroxy-2-[3-(1-adamantyl)-4-hydroxyphényl]benzimidazole. F = 307-309°C.

### EXEMPLE 2 : 5-Hydroxy-2-[3-(1-adamantyl)-4-hydroxyphényl]benzimidazole

### A - Procédé A

### a) 4-méthoxy-2-nitro N-[3-(1-adamantyl)-4-méthoxybenzoyl]aniline

Dans un ballon, on introduit 5,38 g (32 mM) de 2-nitro-4-méthoxyaniline et 50 ml de pyridine. Puis on ajoute goutte à goutte 9,7 g (32 mM) de chlorure de l'acide 3-(1-adamantyl)-4-méthoxy benzoïque solubilisé dans 100 ml de dichlorométhane. En fin d'addition, le mélange est porté à reflux pendant 12 heures. Après avoir refroidi, on ajoute 100 ml d'hexane et le précipité obtenu est récupéré par filtration et lavé par un mélange d'éther 10 % et d'hexane 90 % puis par de l'eau. Après séchage, on obtient 12,5 g (89 %) de produit attendu. F = 208-210°C.

### b) 4-méthoxy-2-amino N-[3-(1-adamantyl)-4-méthoxybenzoyl]aniline

Dans un ballon, on introduit 11,3 g (25,9 mM) de dérivé nitré préparé ci-dessus, 110 ml d'éthanol et 26,5 g (140 mM) de dichlorure d'étain. Le milieu est porté à reflux pendant 2 heures, puis évaporé à sec. Le résidu est repris par 200 ml d'acétate d'éthyle et 100 ml d'eau et 20 ml de soude à 40%. La phase organique est décantée, lavée par une solution aqueuse saturée de carbonate di-sodique et séchée sur sulfate de magnésium. Après évaporation à sec et lavage du résidu par un mélange d'éther 10 % et d'hexane 90 %, on obtient 6,6 g (62 %) de produit correspondant qui est utilisé tel quel pour la suite de la synthèse. F = 211-213°C.

### c) 5-méthoxy-2-[3-(1-adamantyl)-4-méthoxyphényl]benzimidazole

De manière analogue à l'exemple 1(g), à partir de 6,49 g d'amide précédent, on obtient, après lavage du produit brut à l'éther, 5,8 g (93%) de 5-méthoxy-2-[3-(1-adamantyl)-4-méthoxyphényl]benzimidazole de point de fusion F = 148-150°C.

### d) 5-hydroxy-2-[3-(1-adamantyl)-4-hydroxyphényl]benzimidazole

Dans un ballon muni d'une agitation, d'un réfrigérant et d'une circulation d'azote, on introduit 5,5 g (14,2 mM) du produit préparé en (c) et 25 g de chlorhydrate de pyridine. Le milieu réactionnel est chauffé à 200°C pendant 3 heures. Après avoir refroidi, le mélange est jeté dans l'eau et après neutralisation par de la lessive de soude, le précipité obtenu est filtré puis lavé à l'eau. Le produit est chromatographié sur colonne de silice (éluant : THF 60 % et hexane 40 %) puis recristallisé dans le dioxanne, on obtient 1,4 g (27 %) de 5-hydroxy-2-[3-(1-adamantyl)-4-hydroxyphényl]benzimidazole. F = 241-243°C.

### B - Procédé B

3,5 g (6,48 mM) du dérivé obtenu ci-après à l'exemple 13, sont hydrogénés dans les conditions décrites à l'exemple 4, pour conduire après le même traitement, à 1,49 g (64 %) de 5-Hydroxy-2-[3-(1-adamantyl)-4-hydroxyphényl] benzimidazole, de point de fusion 240-245°C.

### EXEMPLE 3

### 5-Benzyloxy-2-[3-(1-adamantyl)-4-méthoxyphényl]benzimidazole

### a) 2-Nitro-4-benzyloxyaniline

28,87 g (187,5 mM) de 2-Nitro-4-hydroxyaniline dans 600 ml de méthyléthylcétone (MEC) sont traités par 77,6 g de carbonate de potassium et 22,3 ml (187,5 mM) de bromure de benzyle. Le milieu réactionnel est chauffé à reflux pendant 3 h, puis est évaporé à sec. Le résidu d'évaporation est trituré dans l'hexane, est filtré et séché sous vide. On isole ainsi 40 g (88,9%) du dérivé attendu, de point de fusion 106°C.

### b) 2-Amino-4-benzyloxyaniline

48,8 g du dérivé nitré obtenu à l'exemple 3(a) sont placés dans 300 ml d'éthanol à 95%. On ajoute à cette solution 16 ml d'hydroxyde de sodium à 20% dans l'eau. Le mélange réactionnel est chauffé à reflux, puis on ajoute 52,3 g (800 mM) de zinc en poudre. La réaction est chauffée à reflux 4 h. On ajoute 1,8 g de hydrosulfite de sodium, puis filtre à chaud et évapore à sec. Le résidu d'évaporation est chromatographié sur silice dans l'éluant dichlorométhane/acétate d'éthyle (70:30) pour conduire à 15,7 g (36,7%) du dérivé attendu, de point de fusion 184°C.

### c) 4-Benzyloxy-2-amino N-[3-(1-adamantyl)-4-méthoxybenzoyl]aniline

6,41 g (20 mM) du chlorure de l'acide 3-(1-Adamantyl)-4-méthoxybenzoïque obtenu selon les conditions décrites pour la préparation de 1(e), sont mis en solution dans 60 ml de THF sec et sont ajoutés goutte-à-goutte à une solution constituée par 4,28 g (20 mM) de l'amine obtenue à l'exemple 3(b). La réaction est laissée sous agitation à température ambiante une nuit. Après le même traitement qu'à l'exemple 1(f) suivi d'une chromatographie sur silice dans le mélange acétate d'éthyle/hexane (40:60), on isole 7,2 g (74,7%) du dérivé attendu, de point de fusion 185°C.

### d) 5-Benzyloxy-2-[3-(1-adamantyl)-4-méthoxyphényl]benzimidazole

5,78 g (12 mM) de l'amide obtenue à l'exemple 3(c) sont placés dans 60 ml de toluène et sont traités par 1,12 ml (12 mM) d'oxychlorure de phosphore, dans les conditions décrites à l'exemple 1(g) pour conduire, après le même traitement, chromatographie sur silice dans l'éluant dichlorométhane/méthanol (99:1) et trituration dans l'éther, à 4,6 g (82,7%) du dérivé attendu, sous forme de produit solide beige.

RMN¹H: δ ppm(CDCl3): 1,65(6H,t,adamantyle) ; 1,93(9H,s,adamantyle) ; 3,82(3H,s,OCH3); 5,01(2H,s,Bn); 6,85-6,94(2H,m,Ar) ; 7,10-7,42(7H,m,Ar) ; 7,86-7,91(2H,d,Ar) ; 11,29(1H,NH).

### EXEMPLE 4

### 5-Hydroxy-2-[3-(1-adamantyl)-4-méthoxyphényl]benzimidazole

4,64 g (10 mM) du dérivé obtenu à l'exemple 3, sont placés dans 300 ml d'acide acétique et 300 ml de dioxanne, puis sont hydrogrénés à 60°C, sous une pression d'hydrogène de 7 bars pendant 3 h en présence de 0,93 g de palladium sur charbon (10%). Le milieu réactionnel est filtré sur célite, et évaporé à sec. Le résidu d'évaporation est lavé à l'éther et recristallisé dans du dioxanne, puis décoloré à chaud par du charbon noir animal. On isole 2,6 g du dérivé attendu, de point de fusion 229,8°C.

### EXEMPLE 5

### 5-Benzyloxy-2-[3-(1-méthylcyclohexyl)-4-benzyloxyphényl]benzimidazole

### a) Acide 3-(1-méthylcyclohexyl)-4-benzyloxybenzoïque

17,48 g (65 mM) de 2-(1-méthylcyclohexyl)-4-bromophenol sont placés dans 200 ml de MEC et sont traités par 27 g (195 mM) de carbonate de potassium. On ajoute ensuite au mélange porté à reflux, 8,5 g (71,5 mM) de bromure de benzyle et continue à chauffer à reflux pendant 4 h. Le mélange réactionnel est filtré. Après évaporation du filtrat, le résidu est chromatographié sur silice dans l'hexane pour conduire à 16,4 g (70,4%) de 3-(1-méthylcyclohexyl)-4-benzyloxybromobenzène, sous forme d'huile incolore.

Une solution de 16,15 g (45 mM) de ce dérivé bromé, dans 80 ml de THF sec, est ajoutée goutte-à-goutte dans un tricol, sous azote, contenant 1,2 g (49,5 mM) de magnésium, ainsi qu'un cristal d'iode. Un léger reflux est maintenu pendant l'addition, puis 1 h après la fin de l'addition. Le mélange réactionnel est refroidi à -40°C puis est soumis à un bullage de gaz carbonique. La réaction est versée dans l'eau, acidifiée par 15 ml d'HCl 5N. On extrait par l'acétate d'éthyle, rince à l'eau jusqu'à pH 7, sèche sur sulfate de magnésium et évapore. Après recristallisation dans le mélange hexane/éther, on isole 7,6 g (52,1%) du dérivé attendu, de point de fusion 157°C.

### b) 5-Benzyloxy-2-[3-(1-méthylcyclohexyl)-4-benzyloxyphényl]benzimidazole

4,08 g (12,6 mM) de l'acide obtenu à l'exemple 5(a) et 3,23 g (15 mM) de 2-Amino-4-benzyloxyaniline sont placés dans 50 ml d'orthodichlorobenzène. On ajoute alors 50 mg de chlorure d'aluminium, chauffe à 130°C et ajoute ensuite goutte-à-goutte 1,54 ml (17,64 mM) de trichlorure de phosphore. Le milieu réactionnel est chauffé à 130°C pendant 2 h, puis est versé dans l'eau et neutralisé par addition d'une solution d'hydroxyde de sodium 2N. On extrait par l'acétate d'éthyle, rince la phase organique à l'eau, sèche sur sulfate de magnésium et évapore. Le résidu est chromatographié sur silice dans l'éluant dichlorométhane/méthanol (99:1) et recristallisé dans le mélange éthanol/eau pour conduire à 2,7 g (42,7%) du dérivé attendu, de point de fusion 209°C.

### EXEMPLE 6

### 5-Hydroxy-2-[3-(1-méthylcyclohexyl)-4-hydroxyphényl]benzimidazole

2,68 g (5,35 mM) du composé obtenu à l'exemple 5(b) sont hydrogénés dans les conditions décrites à l'exemple 4. On isole, après chromatographie sur silice dans l'éluant dichlorométhane/méthanol (92:8), 1,3 g (75%) du dérivé attendu, de point de fusion 180-185°C.

### EXEMPLE 7

### 5-méthoxy-2-[3-(1-adamantyl)-4-benzyloxyphényl]benzimidazole

### a) 3-(1-Adamantyl)-4-benzyloxybenzoate de méthyle

57 g (200 mM) de 3-(1-Adamantyl)-4-hydroxybenzoate de méthyle sont traités par du bromure de benzyle dans les conditions décrites à l'exemple 5(a), pour conduire à 69,5 g (92,4%) du dérivé attendu, de point de fusion 127°C.

### b) Acide 3-(1-Adamantyl)-4-benzyloxybenzoïque

69,5 g (184,8 mM) de l'ester obtenu à l'exemple 7(a) sont placés dans 600 ml de n-butanol et sont traités par 31,05 g (554,4 mM) d'hydroxyde de potassium. Le mélange est agité à reflux pendant 1 h puis est évaporé. Le résidu d'évaporation est repris dans l'eau, acidifé par 60 ml d'HCl 10M. Le précipité est filtré et rincé à l'eau jusqu'à pH neutre, pour conduire après séchage, à 59,4 g (88,8%) du dérivé attendu, de point de fusion 251°C.

### c) 5-méthoxy-2-[3-(1-adamantyl)-4-benzyloxyphényl]bendmidazole

8,69 g (24 mM) de l'acide obtenu à l'exemple 7(b) et 3,97 g (28,8 mM) de 3,4-diaminoanisole, sont traités dans les conditions décrites à l'exemple 5(b), pour conduire après le même traitement, chromatographie sur silice dans le mélange THF/acétate d'éthyle (40:60) et recristallisation dans le mélange éthanol/eau à 3,3 g (29,7%) du dérivé attendu, de point de fusion 251-253°C.

### EXEMPLE 8

### 5-méthoxy-2-[3-(1-adamantyl)-4-hydroxyphényl]benzimidazole

3,25 g (7 mM) du dérivé obtenu à l'exemple 7(c) sont hydrogénés dans les conditions décrites à l'exemple 4, pour conduire à 1,9 g (72,5%) du dérivé attendu, de point de fusion 315-320°C.

### EXEMPLE 9

### 5-Benzyloxy-2-[3-benzyloxy-4-(1-adamantyl)phényl]benzimidazole

### a) 3-Hydroxy-4-(1-adamantyl)benzoate de méthyle

12,16 g (80 mM) de 3-hydroxybenzoate de méthyle et 12,16 g (80 mM) d'adamantanol, dans 160 ml de dichlorométhane sont traités par une addition goutte-à-goutte de 4,37 ml d'acide sulfurique concentré. Le milieu réactionnel est agité à température ambiante pendant la nuit. Le résidu solide est lavé à l'eau et séché, pour conduire à 18,3 g (79,9%) du dérivé attendu, de point de fusion 244-246°C.

### b) Acide 3-Benzyloxy-4-(1-adamantyl)henzoïque

18 g (63 mM) de l'ester obtenu à l'exemple 9(a) sont traités par du bromure de benzyle dans les conditions décrites à l'exemple 5 (a) pour conduire, après le même traitement, à 17,5 g (74%) de 3-Benzyloxy-4-(1-adamantyl)benzoate de méthyle. Le dérivé obtenu est alors saponifié dans les conditions décrites à l'exemple 7(b) pour conduire, après le même traitement, à 13,6 g (96,6%) du dérivé attendu, de point de fusion 240°C.

### c) 4-Benzyloxy-2-amino N-[3-benzyloxy-4-(1-adamantyl)benzoyl]aniline

2,7 g (12,7 mM) de 2-Amino-4-benzyloxyaniline et 5 g (12,7 mM) du chlorure de l'acide 3-Benzyloxy-4-(ladamantyl)benzoïque sont traités dans les conditions décrites à l'exemple 1(f) pour conduire, après le même traitement et chromatographie sur silice dans l'éluant acétate d'éthyle/hexane (30:70) à 4,2 g (59,3%) du dérivé attendu, de point de fusion 178°C.

### d) 5-Benzyloxy-2-[3-benzyloxy-4-(1-adamantyl)phényl]benzimidazole

4,13 g (7,4 mM) de l'amide obtenu à l'exemple 9(c) sont mis en solution dans 40 ml de xylène et sont traités par 1,55 g (8,14 mM) d'acide para-toluènesulfonique. Le milieu réactionnel est chauffé à reflux une nuit, puis est versé dans l'eau glacée et amené à pH 10 avec du carbonate de potassium. On extrait par l'acétate d'éthyle, rince la phase organique à l'eau, sèche sur sulfate de magnésium et évapore. On obtient, après chromatographie sur silice dans le dichlorométhane 2,7 g (67,5%) du dérivé attendu, de point de fusion 203°C.

### EXEMPLE 10

### 5-Hydroxy-2-[3-hydroxy-4-(1-adamantyl)phényl]benzimidazole

2,5 g (4,63 mM) du dérivé obtenu à l'exemple 9 sont hydrogénés pendant 8 h dans les conditions décrites à l'exemple 4, pour conduire, après le même traitement, suivi d'une chromatographie sur RP 18 (Merck), dans l'éluant méthanol/eau (90:10) à 1,05 g (62,9%) du dérivé attendu, de point de fusion 250-252°C.

### EXEMPLE 11

### 5-Benzyloxy-2-[3-(1-adamantyl)-4-benzyloxy-5-méthoxyphenyl]benzimidazole

### a) 3-(1-Adamantyl)-4-hydroxy-5-méthoxybenzoate de méthyle

12,37 g (68 mM) de 3-méthoxy-4-hydroxybenzoate de méthyle dans 150 ml de dichlorométhane sont traités par 10,33 g (68 mM) d'adamantanol et 3,72 ml d'acide sulfurique concentré, dans les conditions décrites à l'exemple 9(a). Après le même traitement, on isole 20,3 g (94,5%) du dérivé attendu, de point de fusion 179°C.

### b) 3-(1-Adamantyl)-4-benzyloxy-5-méthoxybenzoïque

18,32 g (58 mM) du phénol obtenu à l'exemple 11(a) sont traités par du bromure de benzyle dans les conditions décrites à l'exemple 5(a). Après le même traitement, suivi d'une chromatographie sur silice dans l'éulant hexane/éther (90:10), on isole 15,26 g (59,6%) de 3-(1-Adamantyl)-4-benzyloxy-5-méthoxybenzoate de méthyle. 15,22 g (37,5 mM) de cet ester, dans 150 ml de méthanol sont traités par 6,3 g d'hydroxyde de potassium et sont chauffés à reflux, pendant une nuit. Après le même traitement qu'à l'exemple 1(d), on isole 13,7 g (93,2%) du dérivé attendu, de point de fusion 229°C.

### c) 5-Benzyloxy-2-[3-(1-adamantyl)-4-benzyloxy-5-méthoxyphenyl]benzimidazole

4,94 g (12,6 mM) de l'acide obtenu à l'exemple 11(b) sont traités par un mélange de chlorure d'aluminium et de trichlorure de phosphore, dans les conditions décrites à l'exemple 5(b) pour conduire après le même traitement, suivi d'une chromatographie sur silice, dans le mélange acétate d'éthyle/hexane (30:70) à 2,6 g (36,2%) du dérivé attendu, de point de fusion 143°C.

### EXEMPLE 12

### 5-Hydroxy-2-[3-(1-adamantyl)-4-hydroxy-5-méthoxyphenyl]benzimidazole

2,39 g (4,2 mM) du dérivé obtenu à l'exemple 11(c) sont hydrogénés dans les conditions décrites à l'exemple 4, pour conduire après le même traitement, suivi d'une chromatographie sur silice dans le mélange dichlorométhane/méthanol (95:5), à 1 g (61%) du dérivé attendu, de point de fusion 227,2°C.

### EXEMPLE 13

### 5-Benzyloxy-2-[3-(1-adamantyl)-4-benzyloxyphenyl]benzimidazole

### a) 2-Amino-4-benzyloxy N-[3-(1-adamantyl)-4-benzyloxybenzoyl]aniline

3,8 g (10 mM) du chlorure d'acide obtenu à l'exemple 1e, et 2,14 g (10 mM) de 2-Amino-4-benzyloxyaniline sont traités dans les conditions décrites à l'exemple 1(f) pour conduire après le même traitement, suivi d'une recristallisation dans l'éthanol, à 4,8 g (87%) du dérivé attendu, de point de fusion 166-170°C.

### b) 5-Benzyloxy-2-[3-(1-adamantyl)-4-benzyloxyphenyl]benzimidazole

4,5 g (8 mM) du dérivé obtenu à l'exemple 13(a) sont traités par de l'oxychlorure de phosphore, dans les conditions décrites à l'exemple 1(g) pour conduire, après le même traitement, suivi d'une recristallisation dans l'éthanol, à 3,67 g (85%) du dérivé attendu, de point de fusion 265-275°C.

### EXEMPLES DE COMPOSITIONS

### EXEMPLE 1 : Crème

On a préparé selon les méthodes usuelles une crème à phase continue aqueuse répondant à la formule suivante :
- 4-Hydroxy-2-[3-(1-adamantyl)-4-hydroxyphényl] benzimidazole 1,00 g
- Alcool cétylique 4,00 g
- Monostéarate de glycérol 2,50 g
- Stéarate de PEG 50 2,50 g
- Vaseline 10,00 g
- Parahydroxybenzoate de méthyle 0,01 g
- Parahydroxybenzoate de propyle 0,01 g
- Eau déminéralisée 79,98 g

### EXEMPLE 2 : Gel

On a préparé selon les méthodes usuelles un gel ayant la composition suivante :
- 5-Hydroxy-2-[3-(1-adamantyl)-4-hydroxyphényl] benzimidazole 2,00 g
- Alcool éthylique 95 % volume 20,00 g
- Hydroxypropyl cellulose 2,00 g
- Eau déminéralisée stérile 76,00 g

### EXEMPLE 3 : Onguent

On a préparé selon les méthodes usuelles un onguent ayant la composition suivante :
- 4-Hydroxy-2-[3-(1-adamantyl)-4-hydroxyphényl] benzimidazole 5,00 g
- Triglycérides des acides caprique et caprylique 49,00 g
- Vaseline 46,00 g

### EXEMPLE 4 : Crème

On a préparé selon les méthodes usuelles une crème à phase continue aqueuse ayant la composition suivante :
- 5-Hydroxy-2-[3-(1-adamantyl)-4-hydroxyphényl] benzimidazole 0,10 g
- Mélange d'alcools de lanoline émulsifs et de cires et huiles raffinées à base d'hydrocarbures vendu sous la dénomination de "Eucérine anhydre" par la Société BDF 40,00 g
- Parahydroxybenzoate de méthyle 0,07 g
- Parahydroxybenzoate de propyle 0,08 g
- Eau déminéralisée 59,75 g

### EXEMPLE 5 : Gélule

On a préparé une poudre ayant la composition suivante :
- 5-Hydroxy-2-[3-(1-adamantyl)-4-hydroxyphényl] benzimidazole 0,125 g
- Amidon de maïs 0,060 g
- Lactose QSP 0,300 g

La poudre obtenue est conditionnée dans une gélule dont la paroi est composée de gélatine, de TiO₂ et d'un conservateur.

## Revendications (Revendications pour l'(les) Etat(s) contractant(s) suivant(s): AT, BE, CH, LI, DE, DK, FR, GB, IT, NL, SE)

1. Dérivés du benzimidazole **caractérisés par le fait qu'**ils répondent à la formule générale suivante : 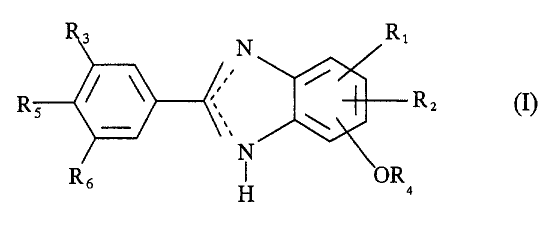 dans laquelle :
R₁ et R₂ représentent un atome d'hydrogène, un radical alkyle ayant de 1 à 6 atomes de carbone, un radical OR₄, un radical fluoroalkyle ayant de 1 à 6 atomes de carbone et de 1 à 5 atomes de fluor ou un atome d'halogène,
R₃ représente un atome d'hydrogène, un radical alkyle ayant de 1 à 6 atomes de carbone, un halogène, un hydroxyle ou un radical alcoxy inférieur ayant de 1 à 6 atomes de carbone,
R₄ représente un atome d'hydrogène, un radical alkyle ayant de 1 à 6 atomes de carbone, un radical benzyle, ou un radical 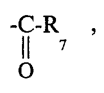 PO₃H, SO₃H ou un reste d'acide aminé,
R₇ représente un radical alkyle ayant de 1 à 6 atomes de carbone, un radical alcoxy inférieur ayant de 1 à 6 atomes de carbone, le radical -(CH₂)ₙ-COOH, n = 1 à 6, ou le radical 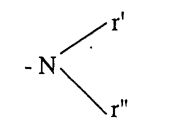
r' et r" représentant un atome d'hydrogène, un radical alkyle ayant de 1 à 6 atomes de carbone, ou r' et r" pris ensemble forment avec l'atome d'azote, un hétérocyle à 5 ou 6 chaînons choisi parmi les radicaux pipéridino, morpholino, pyrrolidino ou pipérazino éventuellement substitué en position 4 par un radical alkyle ayant de 1 à 6 atomes de carbone,
R₅ et R₆, représentent l'un un radical OR₈ et l'autre un radical cycloalkyle mono ou polycyclique ayant de 5 à 12 atomes de carbone, lié au noyau phényle par un carbone tertiaire ;
R₈ représente un atome d'hydrogène, un radical alkyle ayant de 1 à 6 atomes de carbone, un radical acyle ayant de 2 à 7 atomes de carbone, un radical benzyle éventuellement substitué par un ou plusieurs atomes d'halogène ou le radical benzoyle ;
et les sels desdits composés obtenus par addition d'un acide ou d'une base pharmaceutiquement acceptable.

2. Composés selon la revendication 1 **caractérisés par le fait que** le radical alkyle ayant de 1 à 6 atomes de carbone est choisi parmi les radicaux méthyle, éthyle, isopropyle, butyle, 1-méthylpropyle, 1-éthylpropyle, tertiobutyle, 1,1-diméthylpropyle et 1-méthyl 1-éthylpropyle.

3. Composés selon la revendication 1, **caractérisés par le fait que** le radical fluoroalkyle ayant de 1 à 6 atomes de carbone et de 1 à 5 atomes de fluor est de préférence de radical trifluorométhyle.

4. Composés selon la revendication 1, **caractérisés par le fait que** l'atome d'halogène est le chlore, le brome ou le fluor.

5. Composés selon la revendication 1, **caractérisés par le fait que** 1e reste d'acide aminé est un reste dérivant de lysine, de glycine ou de l'acide aspartique.

6. Composés selon la revendication 1, **caractérisés par le fait que** le radical alcoxy est choisi parmi les radicaux méthoxy, éthoxy, isopropoxy ou butoxy.

7. Composés selon la revendication 1, **caractérisés par le fait que** le radical cycloalkyle mono ou polycyclique, ayant de 5 à 12 atomes de carbone, lié au noyau phényle par un carbone tertiaire, est choisi parmi les radicaux 1-adamantyl et 1-méthylcyclohexyle.

8. Composés selon la revendication 1, **caractérisés par le fait que** le radical acyle, ayant de 2 à 7 atomes de carbone, est choisi parmi les radicaux acétyle, propionyle, butyryle, isobutyryle, valéryle ou pivalyle.

9. Composés selon l'une quelconque des revendications précédentes, **caractérisés par le fait qu'**ils sont pris dans le groupe constitué par :
4-hydroxy-2- [3-(1-adamantyl)-4-hydroxyphényl ] benzimidazole,
5-hydroxy-2- [3-(1-adamantyl)-4-hydroxyphényl ] benzimidazole,
5-benzyloxy-2- [ 3-(1-adamantyl)-4-méthoxyphényl ] benzimidazole,
5-hydroxy-2- [3-(1-adamantyl)-4-méthoxyphényl)] benzimidazole,
5-benzyloxy-2- [ 3-(1-méthylcyclohexyl)-4-benzyloxyphényl] benzimidazole,
5-hydroxy-2- [ 3-(1-méthylcyclohexyl)-4-hydroxyphényl) ] benzimidazole,
5-méthoxy-2- [3-(1-adamantyl)-4-benzyloxyphényl ] benzimidazole,
5-méthoxy-2- [ 3-(1-adamantyl)-4-hydroxyphényl ] benzimidazole,
5-benzyloxy-2 [ 3-benzyloxy-4-(1-adamantyl)phényl ] benzimidazole,
5-hydroxy-2- [3-hydroxy-4-(1-adamantyl)phényl ] benzimidazole,
5-benzyloxy-2- [ 3-(1-adamantyl)-4-benzyloxy-5-méthoxyphényl] benzimidazole,
5-hydroxy-2- [3-(1-adamantyl)-4-hydroxy-5-méthoxyphényl ] benzimidazole,
5-benzyloxy-2- [ 3-(1-adamantyl)-4-benzyloxyphényl ] benzimidazole,
5-méthoxy-2- [ 3-(1-adamantyl)-4-méthoxyphényl ] benzimidazole,
5-acétoxy-2- [ 3-(1-adamantyl)-4-acétoxyphényl ] benzimidazole,
5-acétoxy-2- [ 3-(1-adamantyl)-4-hydroxyphényl ] benzimidazole.

10. Procédé de préparation des composés de formule (I) selon l'une quelconque des revendications 1 à 9 et de leurs sels, dans lequel on fait réagir un dérivé d'acide carboxylique aromatique de formule. 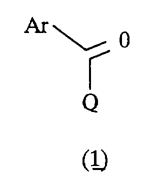 sur une orthonitroaniline de formule : 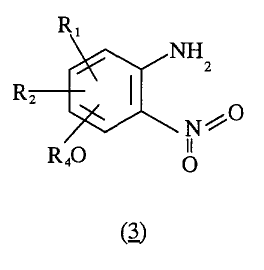 dans lesquels R₁, R₂, et R₄ ont les mêmes significations que celles données à la revendication 1,
Q représente une fonction hydroxy ou un atome de chlore et Ar représente le radical : 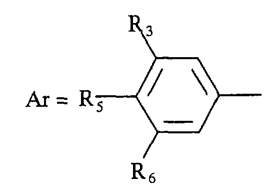 la réaction étant effectuée dans la pyridine, on procède ensuite à une réaction de réduction du composé intermédiaire obtenu puis à une cyclisation par chauffage dans un solvant et en présence d'un réactif choisi parmi l'acide paratoluènesulfonique ou l'oxychlorure de phosphore, et si désiré on prépare les sels desdits composés de formule I par addition d'un acide ou d'une base pharmaceutiquement acceptable.

11. Composition pharmaceutique **caractérisée par le fait qu'**elle contient dans un véhicule approprié pour une administration par voie entérale, parentérale, topique ou oculaire, au moins un composé de formule (I) selon l'une quelconque des revendications 1 à 9.

12. Composition selon la revendication 11, **caractérisée par le fait qu'**elle se présente sous une forme appropriée pour une application topique et contient de 0,005 % à 10 % en poids dudit composé.

13. Utilisation d'un composé selon l'une quelconque des revendications 1 à 9, pour la préparation d'une composition pharmaceutique destinée au traitement ou à la prévention des états inflammatoires et/ou immuno-allergiques.

14. Composition cosmétique destinée à prévenir l'aspect inesthétique de la peau, **caractérisée par le fait qu'**elle contient dans un véhicule cosmétique approprié au moins un composé tel que défini dans l'une quelconque des revendications 1 à 9.

15. Composition cosmétique selon la revendication 14, **caractérisée par le fait qu'**elle contient ledit composé à une concentration comprise entre 0,001 et 5% par rapport au poids total de la composition.

## Revendications (Revendications pour l'(les) Etat(s) contractant(s) suivant(s): ES)

1. Procédé de préparation de dérivés du benzimidazole de la formule générale : 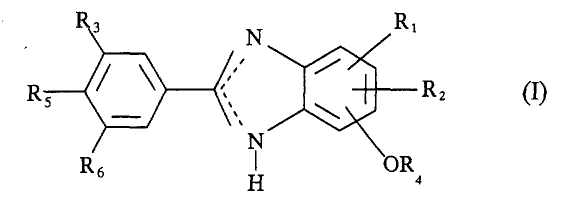 dans laquelle :
R₁ et R₂ représentent un atome d'hydrogène, un radical alkyle ayant de 1 à 6 atomes de carbone, un radical OR₄, un radical fluoroalkyle ayant de 1 à 6 atomes de carbone et de 1 à 5 atomes de fluor ou un atome d'halogène,
R₃ représente un atome d'hydrogène, un radical alkyle ayant de 1 à 6 atomes de carbone, un halogène, un hydroxyle ou un radical alcoxy inférieur ayant de 1 à 6 atomes de carbone,
R₄ représente un atome d'hydrogène, un radical alkyle ayant de 1 à 6 atomes de carbone, un radical benzyle, ou un radical 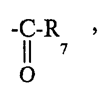 PO₃H, SO₃H ou un reste d'acide aminé,
R₇ représente un radical alkyle ayant de 1 à 6 atomes de carbone, un radical alcoxy inférieur ayant de 1 à 6 atomes de carbone, le radical -(CH₂)ₙ-COOH, n = 1 à 6, ou le radical 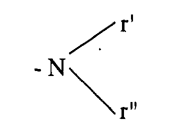
r' et r" représentant un atome d'hydrogène, un radical alkyle ayant de 1 à 6 atomes de carbone, ou r' et r" pris ensemble forment avec l'atome d'azote, un hétérocyle à 5 ou 6 chaînons choisi parmi les radicaux pipéridino, morpholino, pyrrolidino ou pipérazino éventuellement substitué en position 4 par un radical alkyle ayant de 1 à 6 atomes de carbone,
R₅ et R₆, représentent l'un un radical OR₈ et l'autre un radical cycloalkyle mono ou polycyclique ayant de 5 à 12 atomes de carbone, lié au noyau phényle par un carbone tertiaire ;
R₈ représente un atome d'hydrogène, un radical alkyle ayant de 1 à 6 atomes de carbone, un radical acyle ayant de 2 à 7 atomes de carbone, un radical benzyle éventuellement substitué par un ou plusieurs atomes d'halogène ou le radical benzoyle ;
et des sels desdits composés, dans lequel on fait réagir un dérivé d'acide carboxylique aromatique de formule : 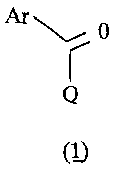 sur une orthonitroaniline de formule : 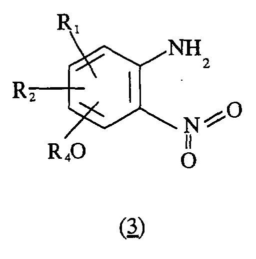 dans lesquels R₁, R₂ et R₄ sont définis comme précédemment,
Q représente une fonction hydroxy ou un atome de chlore et Ar représente le radical : 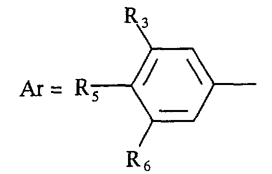 la réaction étant effectuée dans la pyridine, on procède ensuite à une réaction de réduction du composé intermédiaire obtenu puis à une cyclisation par chauffage dans un solvant et en présence d'un réactif choisi parmi l'acide paratoluènesulfonique ou l'oxychlorure de phosphore,
et si désiré on prépare les sels desdits composés de formule I par addition d'un acide ou d'une base pharmaceutiquement acceptable.

2. Procédé selon la revendication 1 **caractérisé par le fait que** le radical alkyle ayant de 1 à 6 atomes de carbone est choisi parmi les radicaux méthyle, éthyle, isopropyle, butyle, 1-méthylpropyle, 1-éthylpropyle, tertiobutyle, 1,1-diméthylpropyle et 1-méthyl 1-éthylpropyle.

3. Procédé selon la revendication 1, **caractérisé par le fait que** le radical fluoroalkyle ayant de 1 à 6 atomes de carbone et de 1 à 5 atomes de fluor est de préférence de radical trifluorométhyle.

4. Procédé selon la revendication 1, **caractérisé par le fait que** l'atome d'halogène est le chlore, le brome ou le fluor.

5. Procédé selon la revendication 1, **caractérisé par le fait que** le reste d'acide aminé est un reste dérivant de lysine, de glycine ou de l'acide aspartique.

6. Procédé selon la revendication 1, **caractérisé par le fait que** le radical alcoxy est choisi parmi les radicaux méthoxy, éthoxy, isopropoxy ou butoxy.

7. Procédé selon la revendication 1, **caractérisé par le fait que** le radical cycloalkyle mono ou polycyclique, ayant de 5 à 12 atomes de carbone, lié au noyau phényle par un carbone tertiaire, est choisi parmi les radicaux 1-adamantyl et 1-méthylcyclohexyle.

8. Procédé selon la revendication 1, **caractérisé par le fait que** le radical acyle, ayant de 2 à 7 atomes de carbone, est choisi parmi les radicaux acétyle, propionyle, butyryle, isobutyryle, valéryle ou pivalyle.

9. Procédé selon l'une quelconque des revendications précédentes, **caractérisé par le fait que** ledit composé est choisi dans le groupe constitué par :
4-hydroxy-2- [3-(1-adamantyl)-4-hydroxyphényl ] benzimidazole,
5-hydroxy-2- [3-(1-adamantyl)-4-hydroxyphényl ] benzimidazole,
5-benzyloxy-2- [ 3-(1-adamantyl)-4-méthoxyphényl ] benzimidazole,
5-hydroxy-2- [3-(1-adamantyl)-4-méthoxyphényl)] benzimidazole,
5-benzyloxy-2- [3-(1-méthylcyclohexyl)-4-benzyloxyphényl] benzimidazole,
5-hydroxy-2- [ 3-(1-méthylcyclohexyl)-4-hydroxyphényl) ] benzimidazole,
5-méthoxy-2- [3-(1-adamantyl)-4-benzyloxyphényl] benzimidazole,
5-méthoxy-2- [ 3-(1-adamantyl)-4-hydroxyphényl ] benzimidazole,
5-benzyloxy-2[3-benzyloxy-4-(1-adamantyl)phényl ] benzimidazole,
5-hydroxy-2- [ 3-hydroxy-4-(1-adamantyl)phényl ] benzimidazole,
5-benzyloxy-2- [ 3-(1-adamantyl)-4-benzyloxy-5-méthoxyphényl] benzimidazole,
5-hydroxy-2- [ 3-(1-adamantyl)-4-hydroxy-5-méthoxyphényl ] benzimidazole,
5-benzyloxy-2- [ 3-(1-adamantyl)-4-benzyloxyphényl] benzimidazole,
5-méthoxy-2- [ 3-(1-adamantyl)-4-méthoxyphényl ] benzimidazole,
5-acétoxy-2- [ 3-(1-adamantyl)-4-acétoxyphényl ] benzimidazole,
5-acétoxy-2- [3-(1-adamantyl)-4-hydroxyphényl ] benzimidazole.

10. Procédé de préparation d'une composition pharmaceutique **caractérisé par le fait que** l'on mélange au moins un composé pouvant être obtenu selon le procédé de l'une quelconque des revendications 1 à 9 avec un véhicule approprié pour une administration par voie entérale, parentérale, topique ou oculaire.

11. Procédé selon la revendication 10, **caractérisé par le fait que** la composition obtenue se présente sous une forme appropriée pour une application topique et contient 0,005 % à 10 % en poids dudit composé.

12. Procédé selon l'une quelconque des revendications 10 et 11 dans lequel la composition pharmaceutique obtenue est destinée au traitement ou à la prévention des états inflammatoires et/ou immuno-allergiques.

13. Composition cosmétique destinée à prévenir l'aspect inesthétique de la peau, **caractérisée par le fait qu'**elle contient dans un véhicule cosmétique approprié au moins un composé pouvant être obtenu selon le procédé de l'une quelconque des revendications 1 à 9.

14. Composition cosmétique selon la revendication 13, **caractérisée par le fait qu'**elle contient ledit composé à une concentration comprise entre 0,001 et 5% par rapport au poids total de la composition.

## Revendications (Revendications pour l'(les) Etat(s) contractant(s) suivant(s): GR)

1. Dérivés du benzimidazole **caractérisés par le fait qu'**ils répondent à la formule générale suivante : 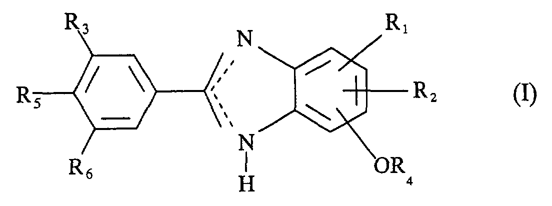 dans laquelle :
R₁ et R₂ représentent un atome d'hydrogène, un radical alkyle ayant de 1 à 6 atomes de carbone, un radical OR₄, un radical fluoroalkyle ayant de 1 à 6 atomes de carbone et de 1 à 5 atomes de fluor ou un atome d'halogène,
R₃ représente un atome d'hydrogène, un radical alkyle ayant de 1 à 6 atomes de carbone, un halogène, un hydroxyle ou un radical alcoxy inférieur ayant de 1 à 6 atomes de carbone,
R₄ représente un atome d'hydrogène, un radical alkyle ayant de 1 à 6 atomes de carbone, un radical benzyle, ou un radical 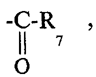 PO₃H, SO₃H ou un reste d'acide aminé,
R₇ représente un radical alkyle ayant de 1 à 6 atomes de carbone, un radical alcoxy inférieur ayant de 1 à 6 atomes de carbone, 1e radical -(CH₂)ₙ-COOH, n = 1 à 6, ou le radical 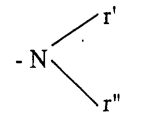
r' et r" représentant un atome d'hydrogène, un radical alkyle ayant de 1 à 6 atomes de carbone, ou r' et r" pris ensemble forment avec l'atome d'azote, un hétérocyle à 5 ou 6 chaînons choisi parmi les radicaux pipéridino, morpholino, pyrrolidino ou pipérazino éventuellement substitué en position 4 par un radical alkyle ayant de 1 à 6 atomes de carbone,
R₅ et R₆, représentent l'un un radical OR₈ et l'autre un radical cycloalkyle mono ou polycyclique ayant de 5 à 12 atomes de carbone, lié au noyau phényle par un carbone tertiaire ;
R₈ représente un atome d'hydrogène, un radical alkyle ayant de 1 à 6 atomes de carbone, un radical acyle ayant de 2 à 7 atomes de carbone, un radical benzyle éventuellement substitué par un ou plusieurs atomes d'halogène ou le radical benzoyle ;
et les sels desdits composés obtenus par addition d'un acide ou d'une base pharmaceutiquement acceptable.

2. Composés selon la revendication 1 **caractérisés par le fait que** le radical alkyle ayant de 1 à 6 atomes de carbone est choisi parmi les radicaux méthyle, éthyle, isopropyle, butyle, 1-méthylpropyle, 1-éthylpropyle, tertiobutyle, 1,1-diméthylpropyle et 1-méthyl 1-éthylpropyle.

3. Composés selon la revendication 1, **caractérisés par le fait que** le radical fluoroalkyle ayant de 1 à 6 atomes de carbone et de 1 à 5 atomes de fluor est de préférence de radical trifluorométhyle.

4. Composés selon la revendication 1, **caractérisés par le fait que** l'atome d'halogène est le chlore, le brome ou le fluor.

5. Composés selon la revendication 1, **caractérisés par le fait que** le reste d'acide aminé est un reste dérivant de lysine, de glycine ou de l'acide aspartique.

6. Composés selon la revendication 1, **caractérisés par le fait que** le radical alcoxy est choisi parmi les radicaux méthoxy, éthoxy, isopropoxy ou butoxy.

7. Composés selon la revendication 1, **caractérisés par le fait que** le radical cycloalkyle mono ou polycyclique, ayant de 5 à 12 atomes de carbone, lié au noyau phényle par un carbone tertiaire, est choisi parmi les radicaux 1-adamantyl et 1-méthylcyclohexyle.

8. Composés selon la revendication 1, **caractérisés par le fait que** le radical acyle, ayant de 2 à 7 atomes de carbone, est choisi parmi les radicaux acétyle, propionyle, butyryle, isobutyryle, valéryle ou pivalyle.

9. Composés selon l'une quelconque des revendications précédentes, **caractérisés par le fait qu'**ils sont pris dans le groupe constitué par :
4-hydroxy-2-[3-(1-adamantyl)-4-hydroxyphényl ] benzimidazole,
5-hydroxy-2-[3-(1-adamantyl)-4-hydroxyphényl ] benzimidazole,
5-benzyloxy-2-[ 3-(1-adamantyl)-4-méthoxyphényl ] benzimidazole,
5-hydroxy-2-[3-(1-adamantyl)-4-méthoxyphényl)] benzimidazole,
5-benzyloxy-2- [ 3-(1-méthylcyclohexyl)-4-benzyloxyphényl] benzimidazole,
5-hydroxy-2- [ 3-(1-méthylcyclohexyl)-4-hydroxyphényl) ] benzimidazole,
5-méthoxy-2- [3-(1-adamantyl)-4-benzyloxyphényl ] benzimidazole,
5-méthoxy-2- [ 3-(1-adamantyl)-4-hydroxyphényl ] benzimidazole,
5-benzyloxy-2 [3-benzyloxy-4-(1-adamantyl)phényl ] benzimidazole,
5-hydroxy-2- [ 3-hydroxy-4-(1-adamantyl)phényl ] benzimidazole,
5-benzyloxy-2-[3-(1-adamantyl)-4-benzyloxy-5-méthoxyphényl ] benzimidazole,
5-hydroxy-2- [ 3-(1-adamantyl)-4-hydroxy-5-méthoxyphényl ]benzimidazole,
5-benzyloxy-2- [ 3-(1-adamantyl)-4-benzyloxyphényl ] benzimidazole,
5-méthoxy-2- [ 3-(1-adamantyl)-4-méthoxyphényl ] benzimidazole,
5-acétoxy-2- [ 3-(1-adamantyl)-4-acétoxyphényl ] benzimidazole,
5-acétoxy-2- [ 3-(1-adamantyl)-4-hydroxyphényl ] benzimidazole.

10. Procédé de préparation des composés de formule (I) selon l'une quelconque des revendications 1 à 9 et de leurs sels, dans lequel on fait réagir un dérivé d'acide carboxylique aromatique de formule. 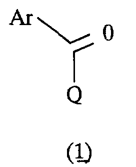 sur une orthonitroaniline de formule : 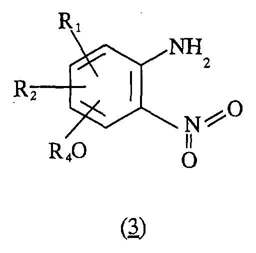 dans lesquels R₁, R₂, et R₄ ont les mêmes significations que celles données à la revendication 1,
Q représente une fonction hydroxy ou un atome de chlore et Ar représente le radical : 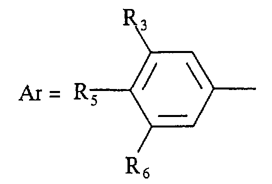 la réaction étant effectuée dans la pyridine, on procède ensuite à une réaction de réduction du composé intermédiaire obtenu puis à une cyclisation par chauffage dans un solvant et en présence d'un réactif choisi parmi l'acide paratoluènesulfonique ou l'oxychlorure de phosphore, et si désiré on prépare les sels desdits composés de Formule I par addition d'un acide ou d'une base pharmaceutiquement acceptable.

11. Procédé de préparation d'une composition pharmaceutique **caractérisé par le fait que** l'on mélange au moins un composé pouvant être obtenu selon le procédé de l'une quelconque des revendications 1 à 9 avec un véhicule approprié pour une administration par voie entérale, parentérale, topique ou oculaire.

12. Procédé selon la revendication 11, **caractérisé par le fait que** la composition obtenue se présente sous une forme appropriée pour une application topique et contient 0,005 % à 10 % en poids dudit composé.

13. Procédé selon l'une quelconque des revendications 11 et 12 dans lequel la composition pharmaceutique obtenue est destinée au traitement ou à la prévention des états inflammatoires et/ou immuno-allergiques.

14. Composition cosmétique destinée à prévenir l'aspect inesthétique de la peau, **caractérisée par le fait qu'**elle contient dans un véhicule cosmétique approprié au moins un composé pouvant être obtenu selon le procédé de l'une quelconque des revendications 1 à 9.

15. Composition cosmétique selon la revendication 14, **caractérisée par le fait qu'**elle contient ledit composé à une concentration comprise entre 0,001 et 5% par rapport au poids total de la composition.

## Patentansprüche (Patentansprüche für folgende(n) Vertragsstaat(en): AT, BE, CH, LI, DE, DK, FR, GB, IT, NL, SE)

1. Benzimidazolderivate, **dadurch gekennzeichnet, daß** diese der folgenden allgemeinen Formel entsprechen: 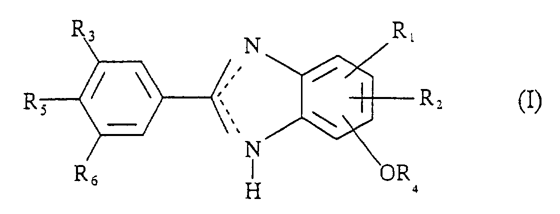 wobei:
R₁ und R₂ ein Wasserstoffatom, einen Alkylrest mit 1-6 Kohlenstoffatomen, einen Rest OR₄, einen Fluoralkylrest mit 1-6 Kohlenstoffatomen und 1-5 Fluoratomen oder ein Halogenatom darstellen,
R₃ ein Wasserstoffatom, einen Alkylrest mit 1-6 Kohlenstoffatomen, ein Halogen, ein Hydroxyl oder einen Niederalkoxyrest mit 1-6 Kohlenstoffatomen darstellt,
R₄ ein Wasserstoffatom, einen Alkylrest mit 1-6 Kohlenstoffatomen, einen Benzylrest oder einen Rest 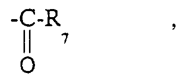 PO₃H, SO₃H oder einen Aminosäurerest darstellt,
R₇ einen Alkylrest mit 1-6 Kohlenstoffatomen, einen Niederalkoxyrest mit 1-6 Kohlenstoffatomen, den Rest -(CH₂)ₙ-COOH, n= 1-6, oder den Rest 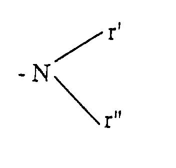 darstellt,
wobei r' und r" ein Wasserstoffatom, einen Alkylrest mit 1-6 Kohlenstoffatomen darstellen, oder r' und r" zusammen mit dem Stickstoffatom einen fünf- oder sechsgliedrigen Heterocyclus bilden, ausgewählt unter den Piperidin-, Morpholin-, Pyrrolidin- oder Piperazin-Resten, gegebenenfalls substituiert in Position 4 mit einem Alkylrest mit 1-6 Kohlenstoffatomen,
R₅ und R₆ zum einen einen Rest OR₈ und zum anderen einen mono- oder polycyclischen Cycloalkylrest mit 5-12 Kohlenstoffatomen, der durch ein tertiäres Kohlenstoffatom an eine Phenylgruppe gebunden ist, darstellen;
R₈ ein Wasserstoffatom, einen Alkylrest mit 1-6 Kohlenstoffatomen, einen Acylrest mit 2-7 Kohlenstoffatomen, einen Benzylrest, der gegebenenfalls mit einem oder mehreren Halogenatomen substituiert ist, oder den Benzoylrest darstellt;
und die Salze dieser Verbindungen, die durch Zugabe einer pharmazeutisch annehmbaren Säure oder Base erhalten werden.

2. Verbindungen nach Anspruch 1, **dadurch gekennzeichnet, daß** der Alkylrest mit 1-6 Kohlenstoffatomen ausgewählt wird unter den Methyl-, Ethyl-, Isopropyl-, Butyl-, 1-Methylpropyl-, 1-Ethylpropyl-, tert-Butyl-, 1,1-Dimethylpropyl- und 1-Methyl-1-ethylpropylresten.

3. Verbindungen nach Anspruch 1, **dadurch gekennzeichnet, daß** der Fluoralkylrest mit 1-6 Kohlenstoffatomen und 1-5 Fluoratomen bevorzugt ein Trifluormethylrest ist.

4. Verbindungen nach Anspruch 1, **dadurch gekennzeichnet, daß** das Halogenatom Chlor, Brom oder Fluor ist.

5. Verbindungen nach Anspruch 1, **dadurch gekennzeichnet, daß** der Aminosäurerest ein Rest ist, der sich von Lysin, Glycin oder Asparaginsäure ableitet.

6. Verbindungen nach Anspruch 1, **dadurch gekennzeichnet, daß** der Alkoxyrest ausgewählt wird unter den Methoxy-, Ethoxy-, Isopropoxy- oder Butoxyresten.

7. Verbindungen nach Anspruch 1, **dadurch gekennzeichnet, daß** der mono- oder polycyclische Cycloalkylrest mit 5-12 Kohlenstoffatomen, der durch ein tertiäres Kohlenstoffatom an eine Phenylgruppe gebunden ist, ausgewählt wird unter den 1-Adamantyl- und 1-Methylcyclohexylresten.

8. Verbindungen nach Anspruch 1, **dadurch gekennzeichnet, daß** der Acylrest mit 2-7 Kohlenstoffatomen ausgewählt wird unter den Acetyl-, Propionyl-, Butyryl-, Isobutyryl-, Valeryl- oder Pivalylresten.

9. Verbindungen nach einem der vorstehenden Ansprüche, **dadurch gekennzeichnet, daß** diese der folgenden Gruppe entnommen werden:
4-Hydroxy-2-[3-(1-adamantyl)-4-hydroxyphenyl]benzimidazol,
5-Hydroxy-2-[3-(1-adamantyl)-4-hydroxyphenyl]benzimidazol,
5-Benzyloxy-2-[3-(1-adamantyl)-4-methoxyphenyl]benzimidazol,
5-Hydroxy-2-[3-(1-adamantyl)-4-methoxyphenyl]benzimidazol,
5-Benzyloxy-2-[3-(1-methylcyclohexyl)-4-benzyloxyphenyl]benzimidazol,
5-Hydroxy-2-[3-(1-methylcyclohexyl)-4-hydroxyphenyl]benzimidazol,
5-Methoxy-2-[3-(1-adamantyl)-4-benzyloxyphenyl]benzimidazol,
5-Methoxy-2-[3-(1-adamantyl)-4-hydroxyphenyl]benzimidazol,
5-Benzyloxy-2[3-benzyloxy-4-(1-adamantyl)phenyl]benzimidazol,
5-Hydroxy-2-[3-hydroxy-4-( 1-adamantyl)phenyl]benzimidazol,
5-Benzyloxy-2-[3-(1-adamantyl)-4-benzyloxy-5-methoxyphenyl]benzimidazol,
5-Hydroxy-2-[3-(1-adamantyl)-4-hydroxy-5-methoxyphenyl]benzimidazol,
5-Benzyloxy-2-[3-(1-adamantyl)-4-benzyloxyphenyl]benzimidazol,
5-Methoxy-2-[3-(1-adamantyl)-4-methoxyphenyl]benzimidazol,
5-Acetoxy-2-[3-(1-adamantyl)-4-acetoxyphenyl]benzimidazol,
5-Acetoxy-2-[3-(1-adamantyl)-4-hydroxyphenyl]benzimidazol.

10. Verfahren zur Herstellung von Verbindungen der Formel (I) nach einem der Ansprüche 1-9 und deren Salzen, bei dem ein aromatisches Carbonsäurederivat der Formel 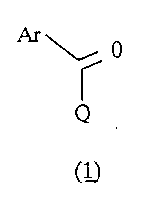 umgesetzt wird mit einem ortho-Nitroanilin der Formel: 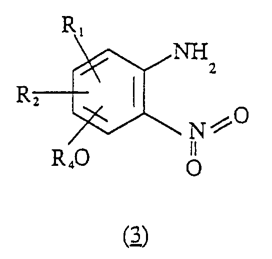 wobei in diesen R₁, R₂ und R₄ die gleichen Bedeutungen aufweisen, wie sie in Anspruch 1 angegeben sind,
Q eine Hydroxyfunktion oder ein Chloratom darstellt und Ar folgenden Rest darstellt: 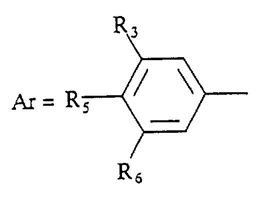 wobei die Reaktion in Pyridin durchgeführt wird, anschließend wird eine Reduktionsreaktion der erhaltenen intermediären Verbindung durchgeführt, dann eine Cyclisierung durch Erwärmen in einem Lösungsmittel und in Anwesenheit eines Reagenz, ausgewählt unter para-Toluolsulfonsäure oder Phosphoroxychlorid, und, falls gewünscht, Cyclisierung durch Erwärmen in einem Lösungsmittel und in Anwesenheit eines Reagenz, ausgewählt unter para-Toluolsulfonsäure oder Phosphoroxychlorid, und, falls gewünscht, werden die Salze der Verbindungen der Formel I durch Zugabe einer pharmazeutisch annehmbaren Säure oder Base hergestellt.

11. Pharmazeutische Zubereitung, **dadurch gekennzeichnet, daß** diese in einem Träger, das für eine enterale, parenterale, topische oder okulare Verabreichung geeignet ist, mindestens eine Verbindung der Formel (I) nach einem der Ansprüche 1-9 enthält.

12. Zubereitung nach Anspruch 11, **dadurch gekennzeichnet, daß** diese in einer für eine topische Anwendung geeigneten Form vorliegt und 0,005 Gew.-% bis 10 Gew.-% der Verbindung enthält.

13. Verwendung einer Verbindung nach einem der Ansprüche 1-9 für die Herstellung einer pharmazeutischen Zubereitung, die für die Behandlung oder die Prävention von Entzündungs- und/oder immunoallergischen Zuständen bestimmt ist.

14. Kosmetische Zubereitung, die dafür bestimmt ist, das unästhetische Aussehen der Haut zu verhindern, **dadurch gekennzeichnet, daß** diese in einem geeigneten kosmetischen Träger mindestens eine Verbindung nach einem der Ansprüche 1-9 enthält.

15. Kosmetische Zubereitung nach Anspruch 14, **dadurch gekennzeichnet, daß** diese die Verbindung in einer Konzentration von 0,001 bis 5 %, bezogen auf das Gesamtgewicht der Zubereitung, enthält.

## Patentansprüche (Patentansprüche für folgende(n) Vertragsstaat(en): ES)

1. Verfahren zur Herstellung von Benzimidazolderivaten der folgenden allgemeinen Formel: 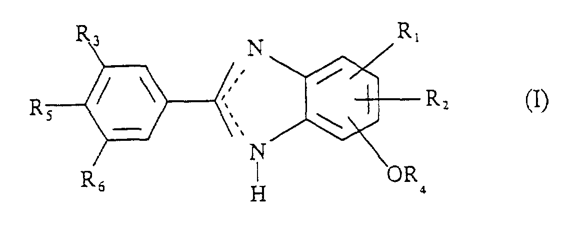 wobei:
R₁ und R₂ ein Wasserstoffatom, einen Alkylrest mit 1-6 Kohlenstoffatomen, einen Rest OR₄, einen Fluoralkylrest mit 1-6 Kohlenstoffatomen und 1-5 Fluoratomen oder ein Halogenatom darstellen,
R₃ ein Wasserstoffatom, einen Alkylrest mit 1-6 Kohlenstoffatomen, ein Halogen, ein Hydroxyl oder einen Niederalkoxyrest mit 1-6 Kohlenstoffatomen darstellt,
R₄ ein Wasserstoffatom, einen Alkylrest mit 1-6 Kohlenstoffatomen, einen Benzylrest oder einen Rest 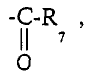 PO₃H, SO₃H oder einen Aminosäurerest darstellt,
R₇ einen Alkylrest mit 1-6 Kohlenstoffatomen, einen Niederalkoxyrest mit 1-6 Kohlenstoffatomen, den Rest -(CH₂)ₙ-COOH, n= 1-6, oder den Rest 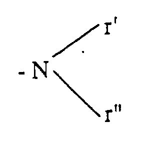 darstellt,
wobei r' und r" ein Wasserstoffatom, einen Alkylrest mit 1-6 Kohlenstoffatomen darstellen, oder r' und r" zusammen mit dem Stickstoffatom einen fünf- oder sechsgliedrigen Heterocyclus bilden, ausgewählt unter den Piperidin-, Morpholin-, Pyrrolidin- oder Piperazin-Resten, gegebenenfalls substituiert in Position 4 mit einem Alkylrest mit 1-6 Kohlenstoffatomen,
R₅ und R₆ zum einen einen Rest OR₈ und zum anderen einen mono- oder polycyclischen Cycloalkylrest mit 5-12 Kohlenstoffatomen, der durch ein tertiäres Kohlenstoffatom an eine Phenylgruppe gebunden ist, darstellen;
R₈ ein Wasserstoffatom, einen Alkylrest mit 1-6 Kohlenstoffatomen, einen Acylrest mit 2-7 Kohlenstoffatomen, einen Benzylrest, der gegebenenfalls mit einem oder mehreren Halogenatomen substituiert ist, oder den Benzoylrest darstellt;
und von Salzen dieser Verbindungen, bei dem ein aromatisches Carbonsäurederivat der Formel: 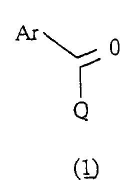 umgesetzt wird mit einem ortho-Nitroanilin der folgenden Formel: 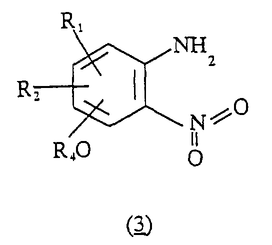 wobei R₁, R₂ und R₄ wie vorstehend definiert sind,
Q eine Hydroxyfunktion oder ein Chloratom darstellt und Ar folgenden Rest darstellt: 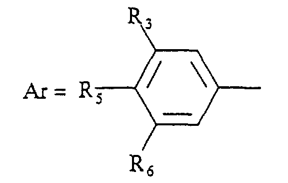 wobei die Reaktion in Pyridin durchgeführt wird, anschließend wird eine Reduktionsreaktion der erhaltenen intermediären Verbindung durchgeführt, dann eine Cyclisierung durch Erwärmen in einem Lösungsmittel und in Anwesenheit eines Reagenz, ausgewählt unter para-Toluolsulfonsäure oder Phosphoroxychlorid,
und, falls gewünscht, werden die Salze der Verbindungen der Formel I durch Zugabe einer pharmazeutisch annehmbaren Säure oder Base hergestellt.

2. Verfahren nach Anspruch 1, **dadurch gekennzeichnet, daß** der Alkylrest mit 1-6 Kohlenstoffatomen ausgewählt wird unter den Methyl-, Ethyl-, Isopropyl-, Butyl-, 1-Methylpropyl-, 1-Ethylpropyl-, tert-Butyl-, 1,1-Dimethylpropyl- und 1-Methyl-1-ethylpropylresten.

3. Verfahren nach Anspruch 1, **dadurch gekennzeichnet, daß** der Fluoralkylrest mit 1-6 Kohlenstoffatomen und 1-5 Fluoratomen bevorzugt der Trifluormethylrest ist.

4. Verfahren nach Anspruch 1, **dadurch gekennzeichnet, daß** das Halogenatom Chlor, Brom oder Fluor ist.

5. Verfahren nach Anspruch 1, **dadurch gekennzeichnet, daß** der Aminosäurerest ein Rest ist, der sich von Lysin, Glycin oder Asparaginsäure ableitet.

6. Verfahren nach Anspruch 1, **dadurch gekennzeichnet, daß** der Alkoxyrest ausgewählt wird unter den Methoxy-, Ethoxy-, Isopropoxy- oder Butoxyresten.

7. Verfahren nach Anspruch 1, **dadurch gekennzeichnet, daß** der mono- oder polycyclische Cycloalkylrest mit 5-12 Kohlenstoffatomen, der über ein tertiäres Kohlenstoffatom an eine Phenylgruppe gebunden ist, ausgewählt wird unter den 1-Adamantyl- und 1-Methylcyclohexylresten.

8. Verfahren nach Anspruch 1, **dadurch gekennzeichnet, daß** der Acylrest mit 2-7 Kohlenstoffatomen ausgewählt wird unter den Acetyl-, Propionyl-, Butyryl-, Isobutyryl-, Valeryl- oder Pivalylresten.

9. Verfahren nach einem der vorstehenden Ansprüche, **dadurch gekennzeichnet, daß** die Verbindung aus der folgenden Gruppe ausgewählt wird:
4-Hydroxy-2-[3-(1-adamantyl)-4-hydroxyphenyl]benzimidazol,
5-Hydroxy-2-[3-(1-adamantyl)-4-hydroxyphenyl]benzimidazol,
5-Benzyloxy-2-[3-(1-adamantyl)-4-methoxyphenyl]benzimidazol,
5-Hydroxy-2-[3-(1-adamantyl)-4-methoxyphenyl]benzimidazol,
5-Benzyloxy-2-[3-(1-methylcyclohexyl)-4-benzyloxyphenyl]benzimidazol,
5-Hydroxy-2-[3-(1-methylcyclohexyl)-4-hydroxyphenyl]benzimidazol,
5-Methoxy-2-[3-(1-adamantyl)-4-benzyloxyphenyl]benzimidazol,
5-Methoxy-2-[3-(1-adamantyl)-4-hydroxyphenyl]benzimidazol,
5-Benzyloxy-2[3-benzyloxy-4-(1-adamantyl)phenyl]benzimidazol,
5-Hydroxy-2-[3-hydroxy-4-(1-adamantyl)phenyl]benzimidazol,
5-Benzyloxy-2-[3-(1-adamantyl)-4-benzyloxy-5-methoxyphenyl]benzimidazol,
5-Hydroxy-2-[3-(1-adamantyl)-4-hydroxy-5-methoxyphenyl]benzimidazol,
5-Benzyloxy-2-[3-(1-adamantyl)-4-benzyloxyphenyl]benzimidazol,
5-Methoxy-2-[3-(1-adamantyl)-4-methoxyphenyl]benzimidazol,
5-Acetoxy-2-[3-(1-adamantyl)-4-acetoxyphenyl]benzimidazol,
5-Acetoxy-2-[3-(1-adamantyl)-4-hydroxyphenyl]benzimidazol.

10. Verfahren zur Herstellung einer pharmazeutischen Zubereitung, **dadurch gekennzeichnet, daß** mindestens eine Verbindung, die gemäß des Verfahrens nach einem der Ansprüche 1-9 erhalten werden kann, mit einem für eine enterale, parenterale, topische oder okulare Verabreichung geeigneten Träger gemischt wird.

11. Verfahren nach Anspruch 10, **dadurch gekennzeichnet, daß** die erhaltene Zubereitung in einer für eine topische Anwendung geeigneten Form vorliegt und 0,005 Gew.-% bis 10 Gew.-% der Verbindung enthält.

12. Verfahren nach einem der Ansprüche 10 und 11, in dem die erhaltene pharmazeutische Zubereitung für die Behandlung oder die Prävention von Entzündungs- und/oder immunoallergischen Zuständen bestimmt ist.

13. Kosmetische Zubereitung, die dafür bestimmt ist, das unästhetische Aussehen der Haut zu verhindern, **dadurch gekennzeichnet, daß** diese in einem geeigneten kosmetischen Vehikel mindestens eine Verbindung enthält, die gemäß des Verfahrens nach einem der Ansprüche 1-9 erhalten werden kann.

14. Kosmetische Zubereitung nach Anspruch 13, **dadurch gekennzeichnet, daß** diese die Verbindung in einer Konzentration von 0,001 bis 5 %, bezogen auf das Gesamtgewicht der Zubereitung, enthält.

## Patentansprüche (Patentansprüche für folgende(n) Vertragsstaat(en): GR)

1. Benzimidazolderivate, **dadurch gekennzeichnet, daß** diese der folgenden allgemeinen Formel entsprechen: 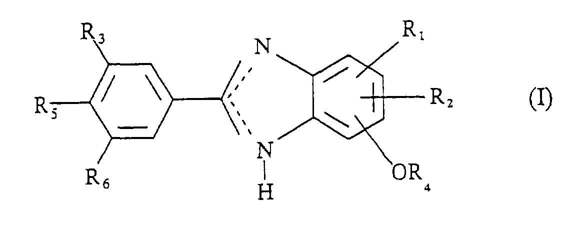 wobei:
R₁ und R₂ ein Wasserstoffatom, einen Alkylrest mit 1-6 Kohlenstoffatomen, einen Rest OR₄, einen Fluoralkylrest mit 1-6 Kohlenstoffatomen und 1-5 Fluoratomen oder ein Halogenatom darstellen,
R₃ ein Wasserstoffatom, einen Alkylrest mit 1-6 Kohlenstoffatomen, ein Halogen, ein Hydroxyl oder einen Niederalkoxyrest mit 1-6 Kohlenstoffatomen darstellt,
R₄ ein Wasserstoffatom, einen Alkylrest mit 1-6 Kohlenstoffatomen, einen Benzylrest oder einen Rest 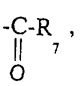 PO₃H, SO₃H oder einen Aminosäurerest darstellt,
R₇ einen Alkylrest mit 1-6 Kohlenstoffatomen, einen Niederalkoxyrest mit 1-6 Kohlenstoffatomen, den Rest -(CH₂)ₙ-COOH, n= 1-6, oder den Rest 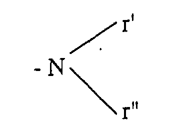 darstellt,
wobei r' und r" ein Wasserstoffatom, einen Alkylrest mit 1-6 Kohlenstoffatomen darstellen, oder r' und r" zusammen mit dem Stickstoffatom einen fünf- oder sechsgliedrigen Heterocyclus bilden, ausgewählt unter den Piperidin-, Morpholin-, Pyrrolidin- oder Piperazin-Resten, gegebenenfalls substituiert in Position 4 mit einem Alkylrest mit 1-6 Kohlenstoffatomen,
R₅ und R₆ zum einen einen Rest OR₈ und zum anderen einen mono- oder polycyclischen Cycloalkylrest mit 5-12 Kohlenstoffatomen, der durch ein tertiäres Kohlenstoffatom an eine Phenylgruppe gebunden ist, darstellen;
R₈ ein Wasserstoffatom, einen Alkylrest mit 1-6 Kohlenstoffatomen, einen Acylrest mit 2-7 Kohlenstoffatomen, einen Benzylrest, der gegebenenfalls mit einem oder mehreren Halogenatomen substituiert ist, oder den Benzoylrest darstellt;
und die Salze dieser Verbindungen, die durch Zugabe einer pharmazeutisch annehmbaren Säure oder Base erhalten werden.

2. Verbindungen nach Anspruch 1, **dadurch gekennzeichnet, daß** der Alkylrest mit 1-6 Kohlenstoffatomen ausgewählt wird unter den Methyl-, Ethyl-, Isopropyl-, Butyl-, 1-Methylpropyl-, 1-Ethylpropyl-, tert-Butyl-, 1,1-Dimethylpropyl- und 1-Methyl-1-ethylpropylresten.

3. Verbindungen nach Anspruch 1, **dadurch gekennzeichnet, daß** der Fluoralkylrest mit 1-6 Kohlenstoffatomen und 1-5 Fluoratomen bevorzugt ein Trifluormethylrest ist.

4. Verbindungen nach Anspruch 1, **dadurch gekennzeichnet, daß** das Halogenatom Chlor, Brom oder Fluor ist.

5. Verbindungen nach Anspruch 1, **dadurch gekennzeichnet, daß** der Aminosäurerest ein Rest ist, der sich von Lysin, Glycin oder Asparaginsäure ableitet.

6. Verbindungen nach Anspruch 1, **dadurch gekennzeichnet, daß** der Alkoxyrest ausgewählt wird unter den Methoxy-, Ethoxy-, Isopropoxy- oder Butoxyresten.

7. Verbindungen nach Anspruch 1, **dadurch gekennzeichnet, daß** der mono- oder polycyclische Cycloalkylrest mit 5-12 Kohlenstoffatomen, der durch ein tertiäres Kohlenstoffatom an eine Phenylgruppe gebunden ist, ausgewählt wird unter den 1-Adamantyl- und 1-Methylcyclohexylresten.

8. Verbindungen nach Anspruch 1, **dadurch gekennzeichnet, daß** der Acylrest mit 2-7 Kohlenstoffatomen ausgewählt wird unter den Acetyl-, Propionyl-, Butyryl-, Isobutyryl-, Valeryl- oder Pivalylresten.

9. Verbindungen nach einem der vorstehenden Ansprüche, **dadurch gekennzeichnet, daß** diese der folgenden Gruppe entnommen werden:
4-Hydroxy-2-[3-(1-adamantyl)-4-hydroxyphenyl]benzimidazol,
5-Hydroxy-2-[3-(1-adamantyl)-4-hydroxyphenyl]benzimidazol,
5-Benzyloxy-2-[3-(1-adamantyl)-4-methoxyphenyl]benzimidazol,
5-Hydroxy-2-[3-(1-adamantyl)-4-methoxyphenyl]benzimidazol,
5-Benzyloxy-2-[3-(1-methylcyclohexyl)-4-benzyloxyphenyl]benzimidazol,
5-Hydroxy-2-[3-(1-methylcyclohexyl)-4-hydroxyphenyl]benzimidazol,
5-Methoxy-2-[3-(1-adamantyl)-4-benzyloxyphenyl]benzimidazol,
5-Methoxy-2-[3-(1-adamantyl)-4-hydroxyphenyl]benzimidazol,
5-Benzyloxy-2[3-benzyloxy-4-(1-adamantyl)phenyl]benzimidazol,
5-Hydroxy-2-[3-hydroxy-4-(1-adamantyl)phenyl]benzimidazol,
5-Benzyloxy-2-[3-(1-adamantyl)-4-benzyloxy-5-methoxyphenyl]benzimidazol,
5-Hydroxy-2-[3-(1-adamantyl)-4-hydroxy-5-methoxyphenyl]benzimidazol,
5-Benzyloxy-2-[3-(1-adamantyl)-4-benzyloxyphenyl]benzimidazol,
5-Methoxy-2-[3-( 1-adamantyl)-4-methoxyphenyl]benzimidazol,
5-Acetoxy-2-[3-(1-adamantyl)-4-acetoxyphenyl]benzimidazol,
5-Acetoxy-2-[3-(1-adamantyl)-4-hydroxyphenyl]benzimidazol.

10. Verfahren zur Herstellung von Verbindungen der Formel (I) nach einem der Ansprüche 1-9 und deren Salzen, bei dem ein aromatisches Carbonsäurederivat der Formel 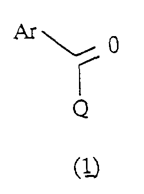 umgesetzt wird mit einem ortho-Nitroanilin der Formel: 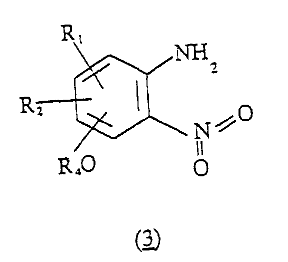 wobei in diesen R₁, R₂ und R₄ die gleichen Bedeutungen aufweisen, wie sie in Anspruch 1 angegeben sind,
Q eine Hydroxyfunktion oder ein Chloratom darstellt und Ar folgenden Rest darstellt: 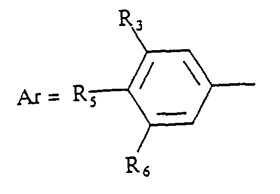 wobei die Reaktion in Pyridin durchgeführt wird, anschließend wird eine Reduktionsreaktion der erhaltenen intermediären Verbindung durchgeführt, dann eine Cyclisierung durch Erwärmen in einem Lösungsmittel und in Anwesenheit eines Reagenz, ausgewählt unter para-Toluolsulfonsäure oder Phosphoroxychlorid, und, falls gewünscht, werden die Salze der Verbindungen der Formel I durch Zugabe einer pharmazeutisch annehmbaren Säure oder Base hergestellt.

11. Verfahren zur Herstellung einer pharmazeutischen Zubereitung, **dadurch gekennzeichnet, daß** mindestens eine Verbindung, die gemäß des Verfahrens nach einem der Ansprüche 1-9 erhalten werden kann, mit einem für eine enterale, parenterale, topische oder okulare Verabreichung geeigneten Vehikel gemischt wird.

12. Verfahren nach Anspruch 11, **dadurch gekennzeichnet, daß** die erhaltene Zubereitung in einer für eine topische Anwendung geeigneten Form vorliegt und 0,005 Gew.-% bis 10 Gew.-% der Verbindung enthält.

13. Verfahren nach einem der Ansprüche 11 und 12, in dem die erhaltene pharmazeutische Zubereitung für die Behandlung oder die Prävention von Entzündungs- und/oder immunoallergischen Zuständen bestimmt ist.

14. Kosmetische Zubereitung, die dafür bestimmt ist, das unästhetische Aussehen der Haut zu verhindern, **dadurch gekennzeichnet, daß** diese in einem geeigneten kosmetischen Vehikel mindestens eine Verbindung nach einem der Ansprüche 1-9 enthält.

15. Kosmetische Zubereitung nach Anspruch 14, **dadurch gekennzeichnet, daß** diese die Verbindung in einer Konzentration von 0,001 bis 5 %, bezogen auf das Gesamtgewicht der Zubereitung, enthält.

## Claims (Claims for the following Contracting State(s): AT, BE, CH, LI, DE, DK, FR, GB, IT, NL, SE)

1. Benzimidazole derivatives, **characterized in that** they correspond to the following general formula: 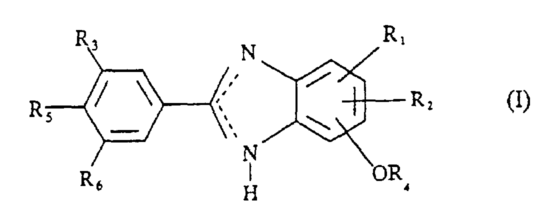 in which:
R₁ and R₂ represent a hydrogen atom, an alkyl radical having from 1 to 6 carbon atoms, an OR₄ radical, a fluoroalkyl radical having from 1 to 6 carbon atoms and from 1 to 5 fluorine atoms or a halogen atom,
R₃ represents a hydrogen atom, an alkyl radical having from 1 to 6 carbon atoms, a halogen, a hydroxyl or a lower alkoxy radical having from 1 to 6 carbon atoms,
R₄ represents a hydrogen atom, an alkyl radical having from 1 to 6 carbon atoms, a benzyl radical or a 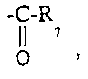 PO₃H or SO₃H radical or an amino acid residue,
R₇ represents an alkyl radical having from 1 to 6 carbon atoms, a lower alkoxy radical having from 1 to 6 carbon atoms, the -(CH₂)ₙ-COOH, n = 1 to 6, radical or the radical 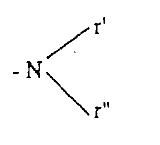
r' and r" representing a hydrogen atom or an alkyl radical having from 1 to 6 carbon atoms or r' and r", taken together, forming, with the nitrogen atom, a 5- or 6-membered heterocycle chosen from the piperidino, morpholino, pyrrolidino or piperazino radicals, the piperazino radical optionally being substituted in the 4-position by an alkyl radical having from 1 to 6 carbon atoms,
one of R₅ and R₆ represents an OR₈ radical and the other a mono- or polycyclic cycloalkyl radical having from 5 to 12 carbon atoms which is bonded to the phenyl nucleus via a tertiary carbon;
R₈ represents a hydrogen atom, an alkyl radical having from 1 to 6 carbon atoms, an acyl radical having from 2 to 7 carbon atoms, a benzyl radical optionally substituted by one or more halogen atoms or the benzoyl radical;
and the salts of the said compounds obtained by addition of a pharmaceutically acceptable acid or base.

2. Compounds according to Claim 1, **characterized in that** the alkyl radical having from 1 to 6 carbon atoms is chosen from the methyl, ethyl, isopropyl, butyl, 1-methylpropyl, 1-ethylpropyl, tert-butyl, 1,1-dimethylpropyl and l-methyl-l-ethylpropyl radicals.

3. Compounds according to Claim 1, **characterized in that** the fluoroalkyl radical having from 1 to 6 carbon atoms and from 1 to 5 fluorine atoms is preferably the trifluoromethyl radical

4. Compounds according to Claim 1, **characterized in that** the halogen atom is chlorine, bromine or fluorine.

5. Compounds according to Claim 1, **characterized in that** the amino acid residue is a residue deriving from lysine, glycine or aspartic acid.

6. Compounds according to Claim 1, **characterized in that** the alkoxy radical is chosen from the methoxy, ethoxy, isopropoxy and butoxy radicals.

7. Compounds according to Claim 1, **characterized in that** the mono- or polycyclic cycloalkyl radical having from 5 to 12 carbon atoms which is bonded to the phenyl nucleus via a tertiary carbon is chosen from the 1-adamantyl and 1-methylcyclohexyl radicals.

8. Compounds according to Claim 1, **characterized in that** the acyl radical having from 2 to 7 carbon atoms is chosen from the acetyl, propionyl, butyryl, isobutyryl, valeryl and pivaloyl radicals.

9. Compounds according to any one of the preceding claims, **characterized in that** they are taken from the group consisting of:
4-hydroxy-2-[3-(1-adamantyl)-4-hydroxyphenyl]benzimidazole,
5-hydroxy-2-[3-(1-adamantyl)-4-hydroxyphenyl]benzimidazole,
5-benzyloxy-2-[3-(1-adamantyl)-4-methoxyphenyl]benzi-midazole,
5-hydroxy-2-[3-(1-adamantyl)-4-methoxyphenyl]benzimidazole,
5-benzyloxy-2-[3-(1-methylcyclohexyl)-4-benzyloxyphenyl]benzimidazole,
5-hydroxy-2-[3-(1-methylcyclohexyl)-4-hydroxyphenyl]benzimidazole,
5-methoxy-2-[3-(1-adamantyl)-4-benzyloxyphenyl]benzimidazole,
5-methoxy-2-[3-(1-adamantyl)-4-hydroxyphenyl]benzimidazole,
5-benzyloxy-2-[3-benzyloxy-4-(1-adamantyl)phenyl]benzimidazole,
5-hydroxy-2-[3-hydroxy-4-(1-adamantyl)phenyl]benzimidazole,
5-benzyloxy-2-[3-(1-adamantyl)-4-benzyloxy-5-methoxyphenyl]benzimidazole,
5-hydroxy-2-[3-(1-adamantyl)-4-hydroxy-5-methoxyphenyl]benzimidazole,
5-benzyloxy-2-[3-(1-adamantyl)-4-benzyloxyphenyl]benzimidazole,
5-methoxy-2-[3-(1-adamantyl)-4-methoxyphenyl]benzimidazole,
5-acetoxy-2-[3-(1-adamantyl)-4-acetoxyphenyl]benzimidazole,
5-acetoxy-2-[3-(1-adamantyl)-4-hydroxyphenyl]benzimidazole.

10. Process for the preparation of the compounds of formula (I) according to any one of Claims 1 to 9 and their salts, in which an aromatic carboxylic acid derivative of formula: 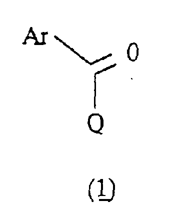 is reacted with an ortho-nitroaniline of formula: 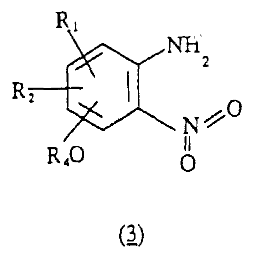 in which R₁, R₂ and R₄ have the same meanings as those given in Claim 1, Q represents a hydroxyl functional group or a chlorine atom and Ar represents the radical: 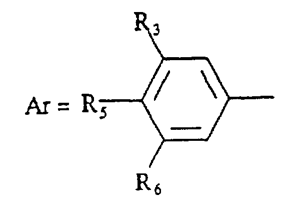 the reaction being carried out in pyridine, a reduction reaction is subsequently carried out on the intermediate compound obtained, then cyclization is carried out by heating in a solvent in the presence of a reactant chosen from paratoluenesulphonic acid or phosphorus oxychloride and, if desired, the salts of the said compounds of formula I are prepared by addition of a pharmaceutically acceptable acid or base.

11. Pharmaceutical composition, **characterized in that** it comprises, in a vehicle appropriate for enteral, parenteral, topical or ocular administration, at least one compound of formula (I) according to any one of Claims 1 to 9.

12. Composition according to Claim 11, **characterized in that** it is provided in a form appropriate for topical application and comprises from 0.005% to 10% by weight of the said compound.

13. Use of a compound according to any one of Claims 1 to 9 for the preparation of a pharmaceutical composition intended for the treatment or prevention of inflammatory and/or immunoallergic conditions.

14. Cosmetic composition intended to prevent an unsightly appearance of the skin, **characterized in that** it comprises, in an appropriate cosmetic vehicle, at least one compound as defined in any one of Claims 1 to 9.

15. Cosmetic composition according to Claim 14, **characterized in that** it comprises the said compound at a concentration of between 0.001 and 5% with respect to the total weight of the composition.

## Claims (Claims for the following Contracting State(s): ES)

1. Process for the preparation of benzimidazole derivatives of the general formula: 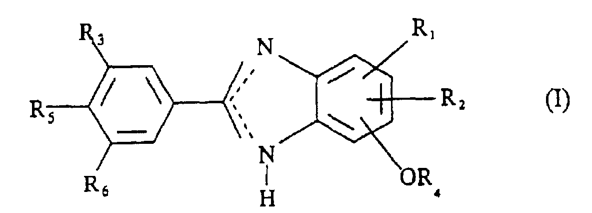 in which:
R₁ and R₂ represent a hydrogen atom, an alkyl radical having from 1 to 6 carbon atoms, an OR₄ radical, a fluoroalkyl radical having from 1 to 6 carbon atoms and from 1 to 5 fluorine atoms or a halogen atom,
R₃ represents a hydrogen atom, an alkyl radical having from 1 to 6 carbon atoms, a halogen, a hydroxyl or a lower alkoxy radical having from 1 to 6 carbon atoms,
R₄ represents a hydrogen atom, an alkyl radical having from 1 to 6 carbon atoms, a benzyl radical or a 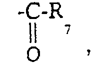 PO₃H or SO₃H radical or an amino acid residue,
R₇ represents an alkyl radical having from 1 to 6 carbon atoms, a lower alkoxy radical having from 1 to 6 carbon atoms the -(CH₂)ₙ-COOH, n = 1 to 6, radical or the radical 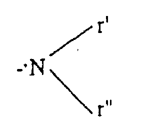
r' and r" representing a hydrogen atom or an alkyl radical having from 1 to 6 carbon atoms or r' and r", taken together, forming, with the nitrogen atom, a 5- or 6-membered heterocycle chosen from the piperidino, morpholino, pyrrolidino or piperazino radicals, the piperazino radical optionally being substituted in the 4-position by an alkyl radical having from 1 to 6 carbon atoms,
one of R₅ and R₆ represents an OR₈ radical and the other a mono- or polycyclic cycloalkyl radical having from 5 to 12 carbon atoms which is bonded to the phenyl nucleus via a tertiary carbon;
R₈ represents a hydrogen atom, an alkyl radical having from 1 to 6 carbon atoms, an acyl radical having from 2 to 7 carbon atoms, a benzyl radical optionally substituted by one or more halogen atoms or the benzoyl radical;
and the salts of the said compounds, in which an aromatic carboxylic acid derivative of formula: 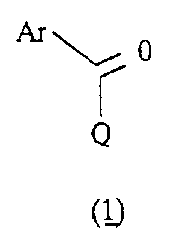 is reacted with an ortho-nitroaniline of formula: 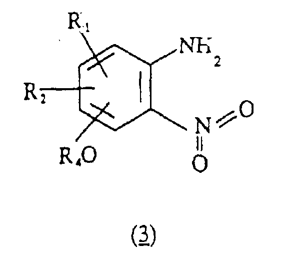 in which R₁, R₂ and R₄ are defined as above, Q represents a hydroxyl functional group or a chlorine atom and Ar represents the radical: 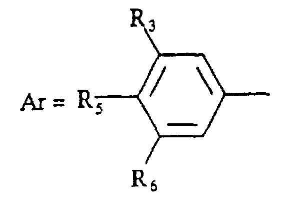 the reaction being carried out in pyridine, a reduction reaction is subsequently carried out on the intermediate compound obtained, then cyclization is carried out by heating in a solvent in the presence of a reactant chosen from paratoluenesulphonic acid or phosphorus oxychloride and, if desired, the salts of the said compounds of formula I are prepared by addition of a pharmaceutically acceptable acid or base.

2. Process according to Claim 1, **characterized in that** the alkyl radical having from 1 to 6 carbon atoms is chosen from the methyl, ethyl, isopropyl, butyl, 1-methylpropyl, 1-ethylpropyl, tert-butyl, 1,1-dimethylpropyl and 1-methyl-l-ethylpropyl radicals.

3. Process according to Claim 1, **characterized in that** the fluoroalkyl radical having from 1 to 6 carbon atoms and from 1 to 5 fluorine atoms is preferably the trifluoromethyl radical.

4. Process according to Claim 1, **characterized in that** the halogen atom is chlorine, bromine or fluorine.

5. Process according to Claim 1, **characterized in that** the amino acid residue is a residue deriving from lysine, glycine or aspartic acid.

6. Process according to Claim 1, **characterized in that** the alkoxy radical is chosen from the methoxy, ethoxy, isopropoxy and butoxy radicals.

7. Process according to Claim 1, **characterized in that** the mono- or polycyclic cycloalkyl radical having from 5 to 12 carbon atoms which is bonded to the phenyl nucleus via a tertiary carbon is chosen from the 1-adamantyl and 1-methylcyclohexyl radicals.

8. Process according to Claim 1, **characterized in that** the acyl radical having from 2 to 7 carbon atoms is chosen from the acetyl, propionyl, butyryl, isobutyryl, valeryl and pivaloyl radicals.

9. Process according to any one of the preceding claims, **characterized in that** the said compound is chosen from the group consisting of:
4-hydroxy-2-[3-(1-adamantyl)-4-hydroxyphenyl]benzimidazole,
5-hydroxy-2-[3-(1-adamantyl)-4-hydroxyphenyl]benzimidazole,
5-benzyloxy-2-[3-(1-adamantyl)-4-methoxyphenyl]benzimidazole,
5-hydroxy-2-[3-(1-adamantyl)-4-methoxyphenyl]benzimidazole,
5-benzyloxy-2-[3-(1-methylcyclohexyl)-4-benzyloxyphenyl]benzimidazole,
5-hydroxy-2-[3-(1-methylcyclohexyl)-4-hydroxyphenyl]benzimidazole,
5-methoxy-2-[3-(1-adamantyl)-4-benzyloxyphenyl]benzimidazole,
5-methoxy-2-[3-(1-adamantyl)-4-hydroxyphenyl]benzimidazole,
5-benzyloxy-2-[3-benzyloxy-4-(1-adamantyl)phenyl]benzimidazole,
5-hydroxy-2-[3-hydroxy-4-(1-adamantyl)phenyl]benzimidazole,
5-benzyloxy-2-[3-(1-adamantyl)-4-benzyloxy-5-methoxyphenyl]benzimidazole,
5-hydroxy-2-[3-(1-adamantyl)-4-hydroxy-5-methoxyphenyl]benzimidazole,
5-benzyloxy-2-[3-(1-adamantyl)-4-benzyloxyphenyl]benzimidazole,
5-methoxy-2-[3-(1-adamantyl)-4-methoxyphenyl]benzimidazole,
5-acetoxy-2-[3-(1-adamantyl)-4-acetoxyphenyl]benzimidazole,
5-acetoxy-2-[3-(1-adamantyl)-4-hydroxyphenyl]benzimidazole.

10. Process for the preparation of a pharmaceutical composition, **characterized in that** at least one compound which can be obtained according to the process of any one of Claims 1 to 9 is mixed with a vehicle appropriate for enteral, parenteral, topical or ocular administration.

11. Process according to Claim 10, **characterized in that** the composition obtained is provided in a form appropriate for topical application and comprises 0.005% to 10% by weight of the said compound.

12. Process according to either one of Claims 10 and 11, in which the pharmaceutical composition obtained is intended for the treatment or prevention of inflammatory and/or immunoallergic conditions.

13. Cosmetic composition intended to prevent an unsightly appearance of the skin, **characterized in that** it comprises, in an appropriate cosmetic vehicle, at least one compound which can be obtained according to the process of any one of Claims 1 to 9.

14. Cosmetic composition according to Claim 13, **characterized in that** it comprises the said compound at a concentration of between 0.001 and 5% with respect to the total weight of the composition.

## Claims (Claims for the following Contracting State(s): GR)

1. Benzimidazole derivatives, **characterized in that** they correspond to the following general formula: 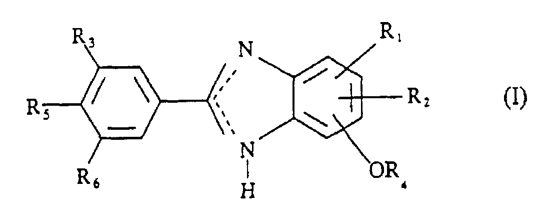 in which:
R₁ and R₂ represent a hydrogen atom, an alkyl radical having from 1 to 6 carbon atoms, an OR₄ radical, a fluoroalkyl radical having from 1 to 6 carbon atoms and from 1 to 5 fluorine atoms or a halogen atom,
R₃ represents a hydrogen atom, an alkyl radical having from 1 to 6 carbon atoms, a halogen, a hydroxyl or a lower alkoxy radical having from 1 to 6 carbon atoms,
R₄ represents a hydrogen atom, an alkyl radical having from 1 to 6 carbon atoms, a benzvl radical or a 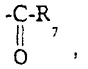 PO₃H or SO₃H radical or an amino acid residue,
R₇ represents an alkyl radical having from 1 to 6 carbon atoms, a lower alkoxy radical having from 1 to 6 carbon atoms, the -(CH₂)ₙ-COOH, n = 1 to 6, radical or the radical 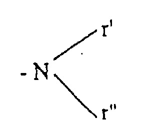
r' and r" representing a hydrogen atom or an alkyl radical having from 1 to 6 carbon atoms or r' and r", taken together, forming, with the nitrogen atom, a 5- or 6-membered heterocycle chosen from the piperidino, morpholino, pyrrolidino or piperazino radicals, the piperazino radical optionally being substituted in the 4-position by an alkyl radical having from 1 to 6 carbon atoms,
one of R₅ and R₆ represents an OR₈ radical and the other a mono- or polycyclic cycloalkyl radical having from 5 to 12 carbon atoms which is bonded to the phenyl nucleus via a tertiary carbon;
R₈ represents a hydrogen atom, an alkyl radical having from 1 to 6 carbon atoms, an acyl radical having from 2 to 7 carbon atoms, a benzyl radical optionally substituted by one or more halogen atoms or the benzoyl radical;
and the salts of the said compounds obtained by addition of a pharmaceutically acceptable acid or base.

2. Compounds according to Claim 1, **characterized in that** the alkyl radical having from 1 to 6 carbon atoms is chosen from the methyl, ethyl, isopropyl, butyl, 1-methylpropyl, 1-ethylpropyl, tert-butyl, 1,1-dimethylpropyl and 1-methyl-l-ethylpropyl radicals.

3. Compounds according to Claim 1, **characterized in that** the fluoroalkyl radical having from 1 to 6 carbon atoms and from 1 to 5 fluorine atoms is preferably the trifluoromethyl radical.

4. Compounds according to Claim 1, **characterized in that** the halogen atom is chlorine, bromine or fluorine.

5. Compounds according to Claim 1, **characterized in that** the amino acid residue is a residue deriving from lysine, glycine or aspartic acid.

6. Compounds according to Claim 1, **characterized in that** the alkoxy radical is chosen from the methoxy, ethoxy, isopropoxy and butoxy radicals.

7. Compounds according to Claim 1, **characterized in that** the mono- or polycyclic cycloalkyl radical having from 5 to 12 carbon atoms which is bonded to the phenyl nucleus via a tertiary carbon is chosen from the 1-adamantyl and 1-methylcyclohexyl radicals.

8. Compounds according to Claim 1, **characterized in that** the acyl radical having from 2 to 7 carbon atoms is chosen from the acetyl, propionyl, butyryl, isobutyryl, valeryl and pivaloyl radicals.

9. Compounds according to any one of the preceding claims, **characterized in that** they are taken from the group consisting of:
4-hydroxy-2-[3-(1-adamantyl)-4-hydroxyphenyl]benzimidazole,
5-hydroxy-2-[3-(1-adamantyl)-4-hydroxyphenyl]benzimidazole,
5-benzyloxy-2-[3-(1-adamantyl)-4-methoxyphenyl]benzimidazole,
5-hydroxy-2-[3-(1-adamantyl)-4-methoxyphenyl]benzimidazole,
5-benzyloxy-2-[3-(1-methylcyclohexyl)-4-benzyloxyphenyl]benzimidazole,
5-hydroxy-2-[3-(1-methylcyclohexyl)-4-hydroxyphenyl]benzimidazole,
5-methoxy-2-[3-(1-adamantyl)-4-benzyloxyphenyl]benzimidazole,
5-methoxy-2-[3-(1-adamantyl)-4-hydroxyphenyl]benzimidazole,
5-benzyloxy-2-[3-benzyloxy-4-(1-adamantyl)phenyl]benzimidazole,
5-hydroxy-2-[3-hydroxy-4-(1-adamantyl)phenyl]benzimidazole,
5-benzyloxy-2-[3-(1-adamantyl)-4-benzyloxy-5-methoxyphenyl]benzimidazole,
5-hydroxy-2-[3-(1-adamantyl)-4-hydroxy-5-methoxyphenyl]benzimidazole,
5-benzyloxy-2-[3-(1-adamantyl)-4-benzyloxyphenyl]benzimidazole,
5-methoxy-2-[3-(1-adamantyl)-4-methoxyphenyl]benzimidazole,
5-acetoxy-2-[3-(1-adamantyl)-4-acetoxyphenyl]benzimidazole,
5-acetoxy-2-[3-(1-adamantyl)-4-hydroxyphenyl]benzimidazole.

10. Process for the preparation of the compounds of formula (I) according to any one of Claims 1 to 9 and their salts, in which an aromatic carboxylic acid derivative of formula: 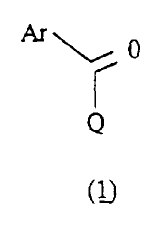 is reacted with an ortho-nitroaniline of formula: 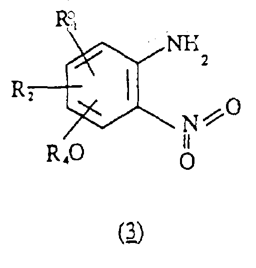 in which R₁, R₂ and R₄ have the same meanings as those given in Claim 1, Q represents a hydroxyl functional group or a chlorine atom and Ar represents the radical: 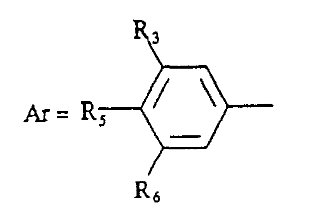 the reaction being carried out in pyridine, a reduction reaction is subsequently carried out on the intermediate compound obtained, then cyclization is carried out by heating in a solvent in the presence of a reactant chosen from paratoluenesulphonic acid or phosphorus oxychloride and, if desired, the salts of the said compounds of formula I are prepared by addition of a pharmaceutically acceptable acid or base.

11. Process for the preparation of a pharmaceutical composition, **characterized in that** at least one compound which can be obtained according to the process of Claim 10 is mixed with a vehicle appropriate for enteral, parenteral, topical or ocular administration.

12. Process according to Claim 11, **characterized in that** the composition obtained is provided in a form appropriate for topical application and comprises 0.005% to 10% by weight of the said compound.

13. Process according to either one of Claims 11 and 12, in which the pharmaceutical composition obtained is intended for the treatment or prevention of inflammatory and/or immunoallergic conditions.

14. Cosmetic composition intended to prevent an unsightly appearance of the skin, **characterized in that** it comprises, in an appropriate cosmetic vehicle, at least one compound which can be obtained according to the process of Claim 10.

15. Cosmetic composition according to Claim 14, **characterized in that** it comprises the said compound at a concentration of between 0.001 and 5% with respect to the total weight of the composition.
